(19) Europäisches Patentamt / European Patent Office / Office européen des brevets

(11) **EP 2 872 126 B1**

(12) **EUROPEAN PATENT SPECIFICATION**

(45) Date of publication and mention of the grant of the patent:
**06.12.2017 Bulletin 2017/49**

(21) Application number: **13737149.8**

(22) Date of filing: **15.07.2013**

(51) Int Cl.:
***A61K 47/10*** *(2017.01)* ***A61K 47/12*** *(2006.01)*
***A61K 9/70*** *(2006.01)* ***A61K 31/137*** *(2006.01)*

(86) International application number:
**PCT/EP2013/002096**

(87) International publication number:
**WO 2014/012653 (23.01.2014 Gazette 2014/04)**

(54) **PHARMACEUTICAL PATCH FOR TRANSDERMAL ADMINISTRATION OF TAPENTADOL**

PHARMAZEUTISCHES PFLASTER ZUR TRANSDERMALEN VERABREICHUNG VON TAPENTADOL

TIMBRE PHARMACEUTIQUE POUR ADMINISTRATION TRANSDERMIQUE DU TAPENTADOL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.07.2012 EP 12005221**
**16.07.2012 US 201261671975 P**

(43) Date of publication of application:
**20.05.2015 Bulletin 2015/21**

(73) Proprietor: **Grünenthal GmbH**
**52078 Aachen (DE)**

(72) Inventors:
- **FRIEDRICH, Ingo**
  **52078 Aachen (DE)**
- **MIKYNA, Martin**
  **81369 München (DE)**
- **GEDAT, Sandra**
  **83043 Bad Aibling (DE)**

(56) References cited:
**US-A1- 2007 298 091**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 872 126 B1

## Description

The invention relates to a pharmaceutical patch for transdermal administration of the pharmacologically active ingredient Tapentadol as defined by the claims, the patch comprising a surface layer, an adhesive layer which comprises a pressure sensitive adhesive and at least a portion of the total amount of the pharmacologically active ingredient that is contained in the pharmaceutical patch, and a removable protective layer, wherein the adhesive layer is located between the surface layer and the removable protective layer.

Tapentadol, the chemical name for which is 3-[(1R,2R)-3-(dimethylamino)-1-ethyl-2-methylpropyl]phenol ((-)-(1R,2R)-3-(3-dimethylamino-1-ethyl-2-methyl-propyl)-phenol), is a synthetic, non-NSAID analgesic that is effective for the treatment of moderate to moderately-severe acute or chronic pain. The compound can be employed as the free base or its physiologically acceptable salts and solvates. Preparation of the free base is known from EP 693 475.

Tapentadol is an investigational, centrally acting analgesic with a dual mode of action consisting of μ-opioid receptor (MOR) agonism and norepinephrine (NE) reuptake inhibition. The efficacy, safety, and pharmacokinetic profile of Tapentadol indicate that the drug may be useful in treating acute as well as chronic pain.Novel and potent Tapentadol dosage forms are disclosed in US 2011/0281855.

While the pharmacologically active ingredient according to the invention has a sufficient bioavailability so that it can be administered orally, it is desirable to provide an alternative route of systemic administration. It is known that transdermal administration of a pharmacologically active ingredient can be advantageous compared to its oral administration, e.g. with respect to bioavailability or patient compliance.

The working principle of a pharmaceutical patch for transdermal administration relies on the release of the pharmacologically active ingredient from the patch, its penetration into and through the skin barrier, and its entry into the systemic circulation through the perfused subcutaneous tissue, where it then develops its pharmacological effect at the targeted receptors. The penetration of a pharmacologically active ingredient through the skin is largely determined by its physicochemical properties and so far, there are only relatively few preparations of pharmacologically active ingredients that are suitable for transdermal administration.

In general, besides the desired pharmacological effect of pain relief, a pharmaceutical patch for transdermal administration of an analgesic should satisfy the following requirements:

- good adhesion to the skin without skin irritations at the contact area, even after long term application;
- appropriate size that is as inconspicuous as possible;
- good shelf-life and storage stability, e.g. no recrystallization of the pharmacologically active ingredient, reduction or even suppression of chemical degradation of the pharmacologically active ingredient;
- low but sufficient content of the pharmacologically active ingredient to maintain therapeutic serum concentrations over extended periods of time; and/or
- well-adjusted flux rate to make available to the patient as much as possible of the pharmacologically active ingredient contained in the pharmaceutical patch over a predetermined period of time at a constant or nearly constant flux rate.

Patent application US 2011/0244022 discloses a composition suitable for use in a transdermal delivery patch for administration of an opioid, the composition comprising a phosphate compound of tocopherol and a polymer carrier.

US20072980941 discloses TTS comprising opioids like tapentadol which can be comprised in the adhesive layer, the adhesive layer Immediately adjoining a carrier layer, wherein the thickness of the adhesive layer is less than 7.0 μm. It is an object of the Invention to provide an advantageous pharmaceutical preparation of Tapentadol as defined by the claims.

This object has been achieved by the subject-matter of the patent claims.

It has been surprisingly found that Tapentadol. In form of the free base or In form of its physiologically acceptable salts, can be administered transdermally. i.e. that pharmaceutical formulations can be found which release the pharmacologically active Ingredient In a form that is capable of penetrating into the skin barrier and passing through the perfused subcutaneous tissue in a sufficient quantity and rate to develop its desired analgesic effect.

Furthermore, It has been surprisingly found that certain pressure sensitive adhesives provide a permeation performance (i.e. flux rate) of Tapentadol through the skin which is superior to other pressure sensitive adhesives. It appears that particularly acrylate-based pressure sensitive adhesives modified with hydroxyl groups provide superior permeation performance of Tapentadol.

A beneficial effect of a specific pressure sensitive adhesive on skin permeation performance depends upon the individual interactions of the pharmacologically active ingredient and the constituents of the pressure sensitive adhesive. Such individual interactions are specific for every pharmacologically active ingredient and cannot be predicted.

The pharmaceutical patch according to the invention comprises a surface layer, an adhesive layer, and a removable protective layer, wherein the adhesive layer is located between the surface layer and the removable protective layer.

The adhesive layer is located between the surface layer and the removable protective layer. Preferably, the surface layer forms the outer surface of the pharmaceutical patch, i.e. when the pharmaceutical patch is applied to the skin the surface layer is the visible layer of the pharmaceutical patch.

Preferably, one of the two opposing surfaces of the adhesive layer is in intimate contact with, i.e. adjacent to the removable protective layer.

In a preferred embodiment, the other of the two opposing surfaces of the adhesive layer is in intimate contact with the surface layer, which in turn preferably forms on its outer surface the outer surface of the pharmaceutical patch. According to this embodiment of the invention, the pharmaceutical patch preferably consists of surface layer, adhesive layer and removable protective layer, so that the adhesive layer contains essentially the total amount of the pharmacologically active ingredient that is contained In the pharmaceutical patch (drug-in-adhesive).

In another preferred embodiment, the other of the two opposing surfaces of the adhesive layer is not in intimate contact with the surface layer, which in turn preferably forms on its outer surface the outer surface of the pharmaceutical patch. Thus, according to this embodiment, at least one additional layer is present between the surface layer and the adhesive layer. According to this embodiment of the invention, the pharmaceutical patch preferably comprises the surface layer, the adhesive layer, the removable protective layer and one, two, three or more additional layers between the adhesive layer and the surface layer, so that at least a portion of the total amount of the pharmacologically active ingredient is present in the adhesive layer (drug-in-adhesive), while the remainder of the pharmacologically active ingredient may be present in any one of said additional layers.

The total thickness of the pharmaceutical patch is not particularly limited. Preferably, the total thickness of the pharmaceutical patch is within the range of from 20 to 1000 μm, more preferably 40 to 800 μm, still more preferably 60 to 650 μm, yet more preferably 80 to 550 μm, most preferably 100 to 450 μm, and in particular 150 to 400 μm. In a preferred embodiment, the total thickness of the pharmaceutical patch is within the range of 100±75 μm (i.e. from 25 μm to 175 μm), preferably 100±50 μm. In another preferred embodiment, the total thickness of the pharmaceutical patch is within the range of 150±100 μm, preferably 150±75 μm, more preferably 150±50 μm. In still another preferred embodiment, the total thickness of the pharmaceutical patch is within the range of 200±150 μm, preferably 200±100 μm, more preferably 200±50 μm. In yet another preferred embodiment, the total thickness of the pharmaceutical patch is within the range of 300±250 μm, preferably 300±200 μm, more preferably 300±150 μm, still more preferably 300±100 μm, and yet more preferably 300±50 μm. In a further preferred embodiment, the total thickness of the pharmaceutical patch is within the range of 400±350 μm, preferably 400±300 μm, more preferably 400±250 μm, still more preferably 400±200 μm, yet more preferably 400±150 μm, even more preferably 400±100 μm, and most preferably 400±50 μm. In still a further preferred embodiment, the total thickness of the pharmaceutical patch is within the range of 500±400 μm, preferably 500±350 μm, more preferably 500±300 μm, still more preferably 500±250 μm, yet more preferably 500±200 μm, even more preferably 500±150 μm, most preferably 500±100 μm, and in particular 500±50 μm. In preferred embodiments, the aforementioned values include the removable protective layer. In another preferred embodiment, the aforementioned values exclude the removable protective layer.

The adhesive layer comprises at least a portion of the total amount of the pharmacologically active ingredient that is contained in the pharmaceutical patch.

Preferably, the adhesive layer comprises at least 10 wt.-%, more preferably at least 25 wt.-%, still more preferably at least 50 wt.-%, yet more preferably at least 75 wt.-%, even more preferably at least 85 wt.-%, most preferably at least 90 wt.-%, and in particular at least 95 wt.-% of the total amount of the pharmacologically active ingredient that is contained in the pharmaceutical patch.

In a preferred embodiment, the adhesive layer is adjacent to the removable protective layer and/or to the surface layer. Preferably, the adhesive layer is adjacent to the removable protective layer and to the surface layer. In a particularly preferred embodiment, the pharmaceutical patch is composed of the surface layer, the adhesive layer, and the removable protective layer and does not contain any additional layer.

In another preferred embodiment, the pharmaceutical patch further comprises at least one drug layer, which comprises at least a portion of the total amount of the pharmacologically active ingredient that is contained in the pharmaceutical patch, i.e. at least a portion of the amount of the pharmacologically active ingredient that is not contained in the adhesive layer.

Preferably, the drug layer comprises at least 10 wt.-%, more preferably at least 25 wt.-%, still more preferably at least 50 wt.-% of the total amount of the pharmacologically active ingredient that is contained in the pharmaceutical patch.

In a preferred embodiment, the drug layer is located between the adhesive layer and the surface layer. The drug layer may be separated from the adhesive layer by a membrane or may be in intimate contact with, i.e. adjacent to the adhesive layer.

In a preferred embodiment, the drug layer comprises a portion of the total amount of the pharmacologically active ingredient and the adhesive layer comprises another portion of the total amount of the pharmacologically active ingredient that is contained in the pharmaceutical patch.

Preferably, when the pharmaceutical patch comprises a drug layer that is separate from the adhesive layer, the drug layer is located between the adhesive layer and the surface layer, in particular adjacent to the adhesive layer. In another preferred embodiment, the drug layer and at least a part of the adhesive layer are both in contact with the same side of the removable protective layer, wherein the area of the drug layer is preferably smaller than the area of the removable protective layer. The adhesive layer may either overlap with the drug layer or be only present in that part of the removable protective layer that is not in contact with the drug layer, for example by forming a ring or a frame around the drug layer.

In a preferred embodiment, the material of the adhesive layer only covers a portion of the adjacent layer(s), e.g. assumes the form of a grid or any other suitable pattern.

The drug layer may be present in form of a liquid, a semisolid, or a solid polymer matrix.

In a preferred embodiment, the drug layer comprises a liquid containing the pharmacologically active ingredient in form of a solution or suspension.

In another preferred embodiment, the drug layer is a semisolid, such as a gel, or a solid polymer matrix wherein the pharmacologically active ingredient is dispersed.

In a preferred embodiment, the total amount of the pharmacologically active ingredient is present in molecular dispersed form.

In another preferred embodiment, only a portion of the pharmacologically active ingredient is present in molecular dispersed form, while the remainder of the pharmacologically active ingredient is present in non-molecular dispersed form (e.g. in form of droplets, crystals and the like) serving the purpose of a depot, also called "microreservoir".

The pharmacologically active ingredient contained in the pharmaceutical patch according to the invention is Tapentadol or a physiologically acceptable salt thereof.

The free base of Tapentadol has the following structural formula (I):

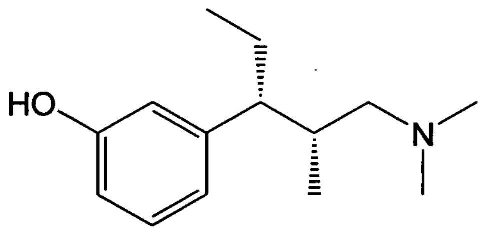

**(I).**

For the purpose of specification, the term "Tapentadol" is intended to include 3-[(1*R*,2*R*)-3-(dimethylamino)-1-ethyl-2-methylpropyl]phenol in form of the free base, i.e. the compound according to formula (I) in any possible form including solvates, cocrystals and polymorphs, and its physiologically acceptable salts, in particular acid addition salts and corresponding solvates, cocrystals and polymorphs.

For the purpose of specification, doses of Tapentadol relate to the free base. Thus, when a physiologically acceptable salt is used instead, its dose has to be adapted to the equivalent dose of the free base. For example, a dose of "200 mg" means an amount of 200 mg of the free base or any equivalent amount of a physiologically acceptable salt or solvate corresponding to 200 mg of the free base (e.g. about 233 mg of the hydrochloride).

The pharmacologically active ingredient can be present in the pharmaceutical patch according to the invention in form of the free base or as derivative thereof in any possible form, thereby particularly including solvates and polymorphs, salts, in particular acid addition salts and corresponding solvates and polymorphs.

The pharmacologically active ingredient can be present in the pharmaceutical patch according to the invention in form of an acid addition salt, whereby any suitable acid capable of forming such an addition salt may be used. The conversion of the pharmacologically active ingredient into a corresponding addition salt, for example, *via* reaction with a suitable acid may be effected in a manner well known to those skilled in the art. Suitable physiologically acceptable salts include salts of inorganic acids, such as hydrochloric acid (Tapentadol HCl), hydrobromic acid and sulfuric acid, and salts of organic acids, such as methane sulfonic acid, fumaric acid, maleic acid, acetic acid, oxalic acid, succinic acid, malic acid, tartaric acid, mandelic acid, lactic acid, citric acid, glutamic acid, acetylsalicylic acid, nicotinic acid, aminobenzoic acid, $\alpha$-liponic acid, hippuric acid and asparaginic acid. The preferred salt is the hydrochloride salt.

Preferably, however, the pharmacologically active ingredient is present in form of the free base. It seems that the transdermal permeability of Tapentadol in form of the free base is higher than the permeability of its hydrochloride addition salt.

In the following, unless expressly stated otherwise, all weight percentages relate to the total weight of the pharmaceutical patch or to the total weight of a specific layer thereof in terms of total per dry unit. In this regard, "dry unit" shall encompass all constituents, irrespective of whether they are present in solid, semisolid or liquid form, but shall

not encompass volatile solvents that are evaporated in course of the preparation of the pharmaceutical patch such as ethanol, heptane, ethyl acetate and the like. Thus, "dry unit" shall merely encompass the residual content of volatile solvent(s), if any.

Preferably, the majority of the pharmacologically active ingredient is contained in the adhesive layer, while a certain portion of the pharmacologically active ingredient may be contained in the adjacent layers e.g. due to migration and/or diffusion.

Preferably, the concentration of the pharmacologically active ingredient in the adhesive layer is in the range of from at least 1.0 to 20.0 wt.-%, more preferably in the range of from at least 2.0 to 17.0 wt.-%, still more preferably in the range of from at least 3.0 to 15.0 wt.-%, most preferably in the range of from at least 4.0 to 13.0 wt.-%, and in particular in the range of from at least 4.5 to 12.0 wt.-%.

In a preferred embodiment, the concentration of the pharmacologically active ingredient in the adhesive layer is at least 1.0 wt.-%, more preferably at least 1.2 wt.-%, still more preferably at least 1.4 wt.-%, yet more preferably at least 1.6 wt.-%, most preferably at least 1.8 wt.-% and in particular at least 2.0 wt.-%, relative to the total weight of the adhesive layer. In another preferred embodiment, the concentration of the pharmacologically active ingredient in the adhesive layer is at least 3.0 wt.-%, more preferably at least 3.2 wt.-%, still more preferably at least 3.4 wt.-%, yet more preferably at least 3.6 wt.-%, even more preferably at least 3.8 wt.-%, and in particular at least 4.0 wt.-%, relative to the total weight of the adhesive layer. In still another preferred embodiment, the concentration of the pharmacologically active ingredient in the adhesive layer is at least 4.0 wt.-%, more preferably at least 4.2 wt.-%, still more preferably at least 4.4 wt.-%, yet more preferably at least 4.6 wt.-%, even more preferably at least 4.8 wt.-%, and in particular at least 5.0 wt.-%, relative to the total weight of the adhesive layer. In yet another preferred embodiment, the concentration of the pharmacologically active ingredient in the adhesive layer is at least 5.0 wt.-%, more preferably at least 5.2 wt.-%, still more preferably at least 5.4 wt.-%, yet more preferably at least 5.6 wt.-%, even more preferably at least 5.8 wt.-%, and in particular at least 6.0 wt.-%, relative to the total weight of the adhesive layer. In a further preferred embodiment, the concentration of the pharmacologically active ingredient in the adhesive layer is at least 7.0 wt.-%, more preferably at least 7.2 wt.-%, still more preferably at least 7.4 wt.-%, yet more preferably at least 7.6 wt.-%, even more preferably at least 7.8 wt.-%, and in particular at least 8.0 wt.-%, relative to the total weight of the adhesive layer. In still a further preferred embodiment, the concentration of the pharmacologically active ingredient in the adhesive layer is at least 9.0 wt.-%, more preferably at least 9.2 wt.-%, still more preferably at least 9.4 wt.-%, yet more preferably at least 9.6 wt.-%, even more preferably at least 9.8 wt.-%, and in particular at least 10.0 wt.-%, relative to the total weight of the adhesive layer. In yet a further preferred embodiment, the concentration of the pharmacologically active ingredient in the adhesive layer is at least 11.0 wt.-%, more preferably at least 11.2 wt.-%, still more preferably at least 11.4 wt.-%, yet more preferably at least 11.6 wt.-%, even more preferably at least 11.8 wt.-%, and in particular at least 12.0 wt.-%, relative to the total weight of the adhesive layer. In another preferred embodiment, the concentration of the pharmacologically active ingredient in the adhesive layer is at least 15.0 wt.-%, more preferably at least 15.2 wt.-%, still more preferably at least 15.4 wt.-%, yet more preferably at least 15.6 wt.-%, even more preferably at least 15.8 wt.-%, and in particular at least 16.0 wt.-%, relative to the total weight of the adhesive layer.

It is principally desirable to provide the pharmacologically active ingredient in the adhesive layer at comparatively high concentrations, as this may positively influence the flux rate. The concentration of the pharmacologically active ingredient in the adhesive layer is preferably at most 75 wt.-% or at most 50 wt.-% or at most 40 wt.-% or at most 30 wt.-% or at most 20 wt.-% or at most 10 wt.-%, more preferably at most 7.5 wt.-%, still more preferably at most 5.0 wt.-%, yet more preferably at most 2.5 wt.-%, even more preferably at most 1.50 wt.-% and in particular at most 1.0 wt.-%, relative to the total weight of the adhesive layer (total per dry unit).

The total dose of the pharmacologically active ingredient that is contained in the pharmaceutical patch is not particularly limited and may depend upon various factors such as body weight of the subject to be treated and duration of application on the skin. The pharmacologically active ingredient is contained in the pharmaceutical patch in a therapeutically effective amount. The amount that constitutes a therapeutically effective amount varies according to the form of the pharmacologically active ingredient being present, the condition being treated, the severity of said condition, the patient being treated, and the prescribed duration of application of the pharmaceutical patch to the skin.

In a preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of $20\pm15$ mg (i.e. from 5 mg to 35 mg), more preferably $20\pm12.5$ mg, still more preferably $20\pm10$ mg, most preferably $20\pm7.5$ mg, and in particular $20\pm5$ mg is systemically administered per day. In still another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of $50\pm40$ mg, more preferably $50\pm35$ mg, still more preferably $50\pm30$ mg, yet more preferably $50\pm25$ mg, even more preferably

50±20 mg, most preferably 50±15 mg, and in particular 50±10 mg is systemically administered per day. In yet another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 75±65 mg, more preferably 75±55 mg, still more preferably 75±45 mg, yet more preferably 75±35 mg, even more preferably 75±25 mg, most preferably 75±20 mg, and in particular 75±10 mg is systemically administered per day. In a further preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 100±90 mg, more preferably 100±80 mg, still more preferably 100±60 mg, yet more preferably 100±50 mg, even more preferably 100±40 mg, most preferably 100±30 mg, and in particular 100±20 mg is systemically administered per day. In still a further preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 150±140 mg, more preferably 150±120 mg, still more preferably 150±100 mg, yet more preferably 150±80 mg, even more preferably 150±60 mg, most preferably 150±40 mg, and in particular 150±20 mg is systemically administered per day. In yet a further preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 200±180 mg, more preferably 200±150 mg, still more preferably 200±120 mg, yet more preferably 200±90 mg, even more preferably 200±70 mg, most preferably 200±40 mg, and in particular 200±20 mg is systemically administered per day. In another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 300±270 mg, more preferably 300±220 mg, still more preferably 300±170 mg, yet more preferably 300±120 mg, even more preferably 300±80 mg, most preferably 300±50 mg, and in particular 300±20 mg is systemically administered per day. In still another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 400±350 mg, more preferably 400±300 mg, still more preferably 400±250 mg, yet more preferably 400±200 mg, even more preferably 400±150 mg, most preferably 400±100 mg, and in particular 400±50 mg is systemically administered per day. In yet another preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 500±450 mg, more preferably 500±350 mg, still more preferably 500±250 mg, yet more preferably 500±150 mg, even more preferably 500±100 mg, most preferably 500±75 mg, and in particular 500±50 mg is systemically administered per day. In a further preferred embodiment, the pharmaceutical patch contains the pharmacologically active ingredient in a quantity so that during 12 hours or during 24 hours or during 48 hours or during 72 hours or during 96 hours or even during 168 hours of consecutive application of a series of pharmaceutical patches to the skin, i.e. under steady state conditions taking into account the depot effect of the skin, an amount of 600±400 mg, more preferably 600±300 mg, still more preferably 600±200 mg, yet more preferably 600±150 mg, even more preferably 600±100 mg, most preferably 600±75 mg, and in particular 600±50 mg is systemically administered per day.

Preferably, the total dose of the pharmacologically active ingredient that is contained in the pharmaceutical patch satisfies the following requirement:

$$\text{dose contained in patch}[\text{mg}] = \frac{\text{intended duration of application}[\text{days}] \cdot \text{desired systemic daily dose}[\text{mg}]}{\text{bioavailability } [\%]} \cdot 100$$

In a preferred embodiment, the desired daily dose (desired systemic daily dose) amounts to 20±15 mg (i.e. from 5 mg to 35 mg), more preferably 20±10 mg; or 50±20 mg, more preferably 50±10 mg; or 75±40 mg, more preferably 75±20 mg; or 100±80 mg, more preferably 100±40 mg; or 150±100 mg, more preferably 150±50 mg; or 200±150 mg, more preferably 200±75 mg; or 300±180 mg, more preferably 300±80 mg; or 400±200 mg, more preferably 400±100 mg; or 500±300 mg, more preferably 500±150 mg; or 600±350 mg, more preferably 600±100

mg. The intended duration of application is preferably 1, 2, 3, 4, 5, 6, or 7 days. The bioavailability is preferably as high as possible and can be determined for a given pharmaceutical patch by routine experimentation.

Preferably, the transdermal bioavailability is within the range of from 1 to 100%.

In a preferred embodiment, the transdermal bioavailability is within the range of 5.0±4.5% (i.e. from 0.5% to 9.5%), more preferably 5.0±4.0%, still more preferably 5.0±3.5%, yet more preferably 5.0±3.0%, even more preferably 5.0±2.5%, most preferably 5.0±2.0%, and in particular 5.0±1.5%. In another preferred embodiment, the transdermal bioavailability is within the range of 10±8%, more preferably 10±7%, still more preferably 10±6%, yet more preferably 10±5%, even more preferably 10±4%, most preferably 10±3%, and in particular 10±2%. In still another preferred embodiment, the transdermal bioavailability is within the range of 30±28%, more preferably 30±25%, still more preferably 30±22%, yet more preferably 30±18%, even more preferably 30±12%, most preferably 30±8%, and in particular 30±5%. In yet another preferred embodiment, the transdermal bioavailability is within the range of 50±45%, more preferably 50±35%, still more preferably 50±25%, yet more preferably 50±15%, even more preferably 50±10%, most preferably 50±8%, and in particular 50±5%. In a further preferred embodiment, the transdermal bioavailability is within the range of 70±65%, more preferably 70±55%, still more preferably 70±45%, yet more preferably 70±35%, even more preferably 70±25%, most preferably 70±15%, and in particular 70±5%. In still a further preferred embodiment, the transdermal bioavailability is within the range of 95±75%, more preferably 95±60%, still more preferably 95±45%, yet more preferably 95±35%, even more preferably 95±25%, most preferably 95±15%, and in particular 95±5%.

Preferably, the area concentration of the pharmacologically active ingredient in the adhesive layer and the drug layer, respectively, is within the range of from 1 to 1,500 $g \cdot m^{-2}$.

In a preferred embodiment, the area concentration of the pharmacologically active ingredient is within the range of 1.00±0.85 $g.m^{-2}$ (i.e. from 0.15 $g.m^{-2}$ to 1.85 $g.m^{-2}$), more preferably 1.00±0.80 $g \cdot m^{-2}$, still more preferably 1.00±0.75 $g \cdot m^{-2}$, yet more preferably 1.00±0.70 $g \cdot m^{-2}$, even more preferably 1.00±0.65 $g \cdot m^{-2}$, most preferably 1.00±0.60 $g \cdot m^{-2}$, and in particular 1.00±0.55 $g \cdot m^{-2}$. In another preferred embodiment, the area concentration of the pharmacologically active ingredient is within the range of 5±4 $g \cdot m^{-2}$, more preferably 5±3 $g \cdot m^{-2}$, still more preferably 5±2 $g \cdot m^{-2}$, most preferably 5±1 $g \cdot m^{2}$, and in particular 5±0.5 $g \cdot m^{-2}$. In still another preferred embodiment, the area concentration of the pharmacologically active ingredient is within the range of 10±8 $g \cdot m^{-2}$, more preferably 10±7 $g \cdot m^{-2}$, still more preferably 10±6 $g \cdot m^{-2}$, yet more preferably 10±5 $g \cdot m^{-2}$, even more preferably 10±4 $g \cdot m^{-2}$, most preferably 10±3 $g \cdot m^{-2}$, and in particular 10±2 $g \cdot m^{-2}$. In yet another preferred embodiment, the area concentration of the pharmacologically active ingredient is within the range of 50±45 $g \cdot m^{-2}$, more preferably 50±35 $g \cdot m^{-2}$, still more preferably 50±25 $g \cdot m^{-2}$, yet more preferably 50±20 $g \cdot m^{-2}$, even more preferably 50±15 $g \cdot m^{-2}$, most preferably 50±10 $g \cdot m^{-2}$, and in particular 50±5 $g \cdot m^{-2}$. In a further preferred embodiment, the area concentration of the pharmacologically active ingredient is within the range of 100±80 $g \cdot m^{-2}$, more preferably 100±60 $g \cdot m^{-2}$, still more preferably 100±50 $g \cdot m^{-2}$, yet more preferably 100±40 $g \cdot m^{-2}$, even more preferably 100±30 $g \cdot m^{-2}$, most preferably 100±20 $g \cdot m^{-2}$, and in particular 100±10 $g \cdot m^{-2}$. In still a further preferred embodiment, the area concentration of the pharmacologically active ingredient is within the range of 150±120 $g \cdot m^{-2}$, more preferably 150±100 $g \cdot m^{-2}$, still more preferably 150±80 $g \cdot m^{-2}$, yet more preferably 150±60 $g \cdot m^{-2}$, even more preferably 150±40 $g \cdot m^{-2}$, most preferably 150±20 $g \cdot m^{-2}$, and in particular 150±15 $g \cdot m^{-2}$. In yet a further preferred embodiment, the area concentration of the pharmacologically active ingredient is within the range of 300±250 $g \cdot m^{-2}$, more preferably 300±220 $g \cdot m^{-2}$, still more preferably 300±200 $g \cdot m^{-2}$, yet more preferably 300±170 $g \cdot m^{-2}$, even more preferably 300±150 $g \cdot m^{-2}$, most preferably 300±120 $g \cdot m^{-2}$, and in particular 300±100 $g \cdot m^{-2}$. In another preferred embodiment, the area concentration of the pharmacologically active ingredient is within the range of 500±400 $g \cdot m^{-2}$, more preferably 500±350 $g \cdot m^{-2}$, still more preferably 500±300 $g \cdot m^{-2}$, yet more preferably 500±250 $g \cdot m^{-2}$, even more preferably 500±200 $g \cdot m^{-2}$, most preferably 500±150 $g \cdot m^{-2}$, and in particular 500±100 $g \cdot m^{-2}$. In still another preferred embodiment, the area concentration of the pharmacologically active ingredient is within the range of 700±600 $g \cdot m^{-2}$, more preferably 700±500 $g \cdot m^{-2}$, still more preferably 700±400 $g \cdot m^{-2}$, yet more preferably 700±350 $g \cdot m^{-2}$, even more preferably 700±300 $g \cdot m^{-2}$, most preferably 700±250 $g \cdot m^{-2}$, and in particular 700±200 $g \cdot m^{-2}$. In yet another preferred embodiment, the area concentration of the pharmacologically active ingredient is within the range of 1,000±850 $g \cdot m^{-2}$, more preferably 1,000±800 $g \cdot m^{-2}$, still more preferably 1,000±750 $g \cdot m^{-2}$, yet more preferably 1,000±700 $g \cdot m^{-2}$, even more preferably 1,000±650 $g \cdot m^{-2}$, most preferably 1,000±600 $g \cdot m^{-2}$, and in particular 1,000±550 $g \cdot m^{-2}$.

Preferably, the pharmaceutical patch upon application to the human skin provides over a period of at least 6 hours, more preferably at least 8 hours, still more preferably at least 10 hours, yet more preferably at least 12 hours, even more preferably at least 18 hours, most preferably at least 24 hours, in particular at least 36 hours release of the pharmacologically active ingredient at a rate in the range of from at least 1.0 to 100,000 $\mu g \cdot cm^{-2} \cdot h^{-1}$.

In a preferred embodiment, the pharmaceutical patch upon application to the human skin provides over a period of at least 6 hours, more preferably at least 12 hours, still more preferably at least 24 hours, release of the pharmacologically active ingredient at a rate of at least 1.0 $\mu g \cdot cm^{-2} \cdot h^{-1}$ or at least 5.0 $\mu g \cdot cm^{-2} \cdot h^{-1}$ or at least 10 $\mu g \cdot cm^{-2} \cdot h^{-1}$; more preferably at least 20 $\mu g \cdot cm^{-2} \cdot h^{-1}$ or at least 40 $\mu g \cdot cm^{-2} \cdot h^{-1}$ or at least 60 $\mu g \cdot cm^{-2} \cdot h^{-1}$; still more preferably at least 80 $\mu g \cdot cm^{-2} \cdot h^{-1}$ or at least 100 $\mu g \cdot cm^{-2} \cdot h^{-1}$ or at least 120 $\mu g \cdot cm^{-2} \cdot h^{-1}$; yet more preferably at least 150 $\mu g \cdot cm^{-2} \cdot h^{-1}$ or at least 250 $\mu g \cdot cm^{-2} \cdot h^{-1}$ or at least 350 $\mu g \cdot cm^{-2} \cdot h^{-1}$; and in particular at least 500 $\mu g \cdot cm^{-2} \cdot h^{-1}$ or at least 1,000 $\mu g \cdot cm^{-2} \cdot h^{-1}$

or at least 10,000 $\mu g \cdot cm^{-2} \cdot h^{-1}$, or at least 100,000 $\mu g \cdot cm^{-2} \cdot h^{-1}$

In a preferred embodiment, the pharmaceutical patch upon application to the human skin provides over a period of at least 6 hours, more preferably at least 12 hours, still more preferably at least 24 hours, release of the pharmacologically active ingredient at a rate of $10\pm8$ $\mu g \cdot cm^{-2} \cdot h^{-1}$ (i.e. from 2 $\mu g \cdot cm^{-2} \cdot h^{-1}$ to 18 $\mu g \cdot cm^{-2} \cdot h^{-1}$), more preferably $10\pm7$ $\mu g \cdot cm^{-2} \cdot h^{-1}$, still more preferably $10\pm6$ $\mu g \cdot cm^{-2} \cdot h^{-1}$, yet more preferably $10\pm5$ $\mu g \cdot cm^{-2} \cdot h^{-1}$, even more preferably $10\pm4$ $\mu g \cdot cm^{-2} \cdot h^{-1}$, most preferably $10\pm3$ $\mu g \cdot cm^{-2} \cdot h^{-1}$ and in particular $10\pm2$ $\mu g \cdot cm^{-2} \cdot h^{-1}$. In another preferred embodiment, the pharmaceutical patch upon application to the human skin provides over a period of at least 6 hours, more preferably at least 12 hours, still more preferably at least 24 hours, release of the pharmacologically active ingredient at a rate of $50\pm45$ $\mu g \cdot cm^{-2} \cdot h^{-1}$, more preferably $50\pm35$ $\mu g \cdot cm^{-2} \cdot h^{-1}$, still more preferably $50\pm30$ $\mu g \cdot cm^{-2} \cdot h^{-1}$, yet more preferably $50\pm25$ $\mu g \cdot cm^{-2} \cdot h^{-1}$, even more preferably $50\pm20$ $\mu g \cdot cm^{-2} \cdot h^{-1}$, most preferably $50\pm15$ $\mu g \cdot cm^{-2} \cdot h^{-1}$ and in particular $50\pm10$ $\mu g \cdot cm^{-2} \cdot h^{-1}$. In still another preferred embodiment, the pharmaceutical patch upon application to the human skin provides over a period of at least 6 hours, more preferably at least 12 hours, still more preferably at least 24 hours, release of the pharmacologically active ingredient at a rate of $100\pm80$ $\mu g \cdot cm^{-2} \cdot h^{-1}$, more preferably $100\pm70$ $\mu g \cdot cm^{-2} \cdot h^{-1}$, still more preferably $100\pm60$ $\mu g \cdot cm^{-2} \cdot h^{-1}$, yet more preferably $100\pm50$ $\mu g \cdot cm^{-2} \cdot h^{-1}$, even more preferably $100\pm40$ $\mu g \cdot cm^{-2} \cdot h^{-1}$, most preferably $100\pm30$ $\mu g \cdot cm^{-2} \cdot h^{-1}$ and in particular $100\pm20$ $\mu g \cdot cm^{-2} \cdot h^{-1}$. In yet another preferred embodiment, the pharmaceutical patch upon application to the human skin provides over a period of at least 6 hours, more preferably at least 12 hours, still more preferably at least 24 hours, release of the pharmacologically active ingredient at a rate of $150\pm100$ $\mu g \cdot cm^{-2} \cdot h^{-1}$, more preferably $150\pm80$ $\mu g \cdot cm^{-2} \cdot h^{-1}$, still more preferably $150\pm60$ $\mu g \cdot cm^{-2} \cdot h^{-1}$, yet more preferably $150\pm50$ $\mu g \cdot cm^{-2} \cdot h^{-1}$, even more preferably $150\pm40$ $\mu g \cdot cm^{-2} \cdot h^{-1}$, most preferably $150\pm30$ $\mu g \cdot cm^{-2} \cdot h^{-1}$ and in particular $150\pm20$ $\mu g \cdot cm^{-2} \cdot h^{-1}$. In a further preferred embodiment, the pharmaceutical patch upon application to the human skin provides over a period of at least 6 hours, more preferably at least 12 hours, still more preferably at least 24 hours, release of the pharmacologically active ingredient at a rate of $300\pm250$ $\mu g \cdot cm^{-2} \cdot h^{-1}$, more preferably $300\pm200$ $\mu g \cdot cm^{-2} \cdot h^{-1}$, still more preferably $300\pm160$ $\mu g \cdot cm^{-2} \cdot h^{-1}$, yet more preferably $300\pm120$ $\mu g \cdot cm^{-2} \cdot h^{-1}$, even more preferably $300\pm100$ $\mu g \cdot cm^{-2} \cdot h^{-1}$, most preferably $300\pm80$ $\mu g \cdot cm^{-2} \cdot h^{-1}$ and in particular $300\pm50$ $\mu g \cdot cm^{-2} \cdot h^{-1}$. In still a further preferred embodiment, the pharmaceutical patch upon application to the human skin provides over a period of at least 6 hours, more preferably at least 12 hours, still more preferably at least 24 hours, release of the pharmacologically active ingredient at a rate of $500\pm450$ $\mu g \cdot cm^{-2} \cdot h^{-1}$, more preferably $500\pm350$ $\mu g \cdot cm^{-2} \cdot h^{-1}$, still more preferably $500\pm300$ $\mu g \cdot cm^{-2} \cdot h^{-1}$, yet more preferably $500\pm250$ $\mu g \cdot cm^{-2} \cdot h^{-1}$, even more preferably $500\pm200$ $\mu g \cdot cm^{-2} \cdot h^{-1}$, most preferably $500\pm150$ $\mu g \cdot cm^{-2} \cdot h^{-1}$ and in particular $500\pm100$ $\mu g \cdot cm^{-2} \cdot h^{-1}$. In yet a further preferred embodiment, the pharmaceutical patch upon application to the human skin provides over a period of at least 6 hours, more preferably at least 12 hours, still more preferably at least 24 hours, release of the pharmacologically active ingredient at a rate of $1{,}000\pm800$ $\mu g \cdot cm^{-2} \cdot h^{-1}$, more preferably $1{,}000\pm700$ $\mu g \cdot cm^{-2} \cdot h^{-1}$, still more preferably $1{,}000\pm600$ $\mu g \cdot cm^{-2} \cdot h^{-1}$, yet more preferably $1{,}000\pm500$ $\mu g \cdot cm^{-2} \cdot h^{-1}$, even more preferably $1{,}000\pm400$ $\mu g \cdot cm^{-2} \cdot h^{-1}$, most preferably $1{,}000\pm300$ and in particular $1{,}000\pm200$ $\mu g \cdot cm^{-2} \cdot h^{-1}$. In another preferred embodiment, the pharmaceutical patch upon application to the human skin provides over a period of at least 6 hours, more preferably at least 12 hours, still more preferably at least 24 hours, release of the pharmacologically active ingredient at a rate of $10{,}000\pm8{,}000$ $\mu g \cdot cm^{-2} \cdot h^{-1}$, more preferably $10{,}000\pm7{,}000$ $\mu g \cdot cm^{-2} \cdot h^{-1}$, still more preferably $10{,}000\pm6{,}000$ $\mu g \cdot cm^{-2} \cdot h^{-1}$, yet more preferably $10{,}000\pm5{,}000$ $\mu g \cdot cm^{-2} \cdot h^{-1}$, even more preferably $10{,}000\pm4{,}000$ $\mu g \cdot cm^{-2} \cdot h^{-1}$, most preferably $10{,}000\pm3{,}000$ $\mu g \cdot cm^{-2} \cdot h^{-1}$ and in particular $10{,}000\pm2{,}000$ $\mu g \cdot cm^{-2} \cdot h^{-1}$. In still another preferred embodiment, the pharmaceutical patch upon application to the human skin provides over a period of at least 6 hours, more preferably at least 12 hours, still more preferably at least 24 hours, release of the pharmacologically active ingredient at a rate of $100{,}000\pm80{,}000$ $\mu g \cdot cm^{-2} \cdot h^{-1}$, more preferably $100{,}000\pm70{,}000$ $\mu g \cdot cm^{-2} \cdot h^{-1}$, still more preferably $100{,}000\pm60{,}000$ $\mu g \cdot cm^{-2} \cdot h^{-1}$, yet more preferably $100{,}000\pm50{,}000$ $\mu g \cdot cm^{-2} \cdot h^{-1}$, even more preferably $100{,}000\pm40{,}000$ $\mu g \cdot cm^{-2} \cdot h^{-1}$, most preferably $100{,}000\pm30{,}000$ $\mu g \cdot cm^{-2} \cdot h^{-1}$ and in particular $100{,}000\pm20{,}000$ $\mu g \cdot cm^{-2} \cdot h^{-1}$.

Low serum concentrations of Tapentadol suffice to show an effect in individuals that are relatively sensitive and higher serum concentrations of Tapentadol are needed to show an effect in persons that are relatively unsensitive. Preliminary clinical trials revealed that a significant pain treating effect is seen at serum concentrations in the range of from about 5 $ng \cdot ml^{-1}$ (approximately -2 mm visual analog scale (VAS) in a population mean) to about 300 $ng \cdot ml^{-1}$ (approximately -15 mm visual analog scale (VAS) in a population mean).

In a preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, within the range of from 0.1 to 10,000 $ng \cdot ml^{-1}$, more preferably 1.0 to 9,000 $ng \cdot ml^{-1}$, still more preferably 2.0 to 8,000 $ng \cdot ml^{-1}$, yet more preferably 3.0 to 5,000 $ng \cdot ml^{-1}$, most preferably 4.0 to 500 $ng \cdot ml^{-1}$ and in particular 5.0 to 300 $ng \cdot ml^{-1}$.

In a preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the

skin, of 10±8 $ng \cdot ml^{-1}$, more preferably 10±7 $ng \cdot ml^{-1}$, still more preferably 10±6 $ng \cdot ml^{-1}$, yet more preferably 10±5 $ng \cdot ml^{-1}$, even more preferably 10±4 $ng \cdot ml^{-1}$, most preferably 10±3 $ng \cdot ml^{-1}$, and in particular 10±2 $ng \cdot ml^{-1}$. In another preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 20±16 $ng \cdot ml^{-1}$, more preferably 20±14 $ng \cdot ml^{-1}$, still more preferably 20±12 $ng \cdot ml^{-1}$, yet more preferably 20±10 $ng \cdot ml^{-1}$, even more preferably 20±8.0 $ng \cdot ml^{-1}$, most preferably 20±6.0 $ng \cdot ml^{-1}$, and in particular 20±4.0 $ng \cdot ml^{-1}$. In still another preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 30±16 $ng \cdot ml^{-1}$, more preferably 30±14 $ng \cdot ml^{-1}$, still more preferably 30±12 $ng \cdot ml^{-1}$, yet more preferably 30±10 $ng \cdot ml^{-1}$, even more preferably 30±8.0 $ng \cdot ml^{-1}$, most preferably 30±7.0 $ng \cdot ml^{-1}$, and in particular 30±6.0 $ng \cdot ml^{-1}$. In yet another preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 40±32 $ng \cdot ml^{-1}$, more preferably 40±28 $ng \cdot ml^{-1}$, still more preferably 40±24 $ng \cdot ml^{-1}$, yet more preferably 40±20 $ng \cdot ml^{-1}$, even more preferably 40±16 $ng \cdot ml^{-1}$, most preferably 40±12 $ng \cdot ml^{-1}$, and in particular 40±8.0 $ng \cdot ml^{-1}$. In a further preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 50±35 $ng \cdot ml^{-1}$, more preferably 50±30 $ng \cdot ml^{-1}$, still more preferably 50±25 $ng \cdot ml^{-1}$, yet more preferably 50±20 $ng \cdot ml^{-1}$, most preferably 50±15 $ng \cdot ml^{-1}$, and in particular 50±10 $ng \cdot ml^{-1}$. In still a further preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 60±45 $ng \cdot ml^{-1}$, more preferably 60±35 $ng \cdot ml^{-1}$, still more preferably 60±30 $ng \cdot ml^{-1}$, yet more preferably 60±25 $ng \cdot ml^{-1}$, even more preferably 60±20 $ng \cdot ml^{-1}$, most preferably 60±15 $ng \cdot ml^{-1}$, and in particular 60±10 $ng \cdot ml^{-1}$. In yet a further preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 75±64 $ng \cdot ml^{-1}$, more preferably 75±56 $ng \cdot ml^{-1}$, still more preferably 75±48 $ng \cdot ml^{-1}$, yet more preferably 75±40 $ng \cdot ml^{-1}$, even more preferably 75±32 $ng \cdot ml^{-1}$, most preferably 75±24 $ng \cdot ml^{-1}$, and in particular 75±16 $ng \cdot ml^{-1}$. In another preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 90±60 $ng \cdot ml^{-1}$, more preferably 90±50 $ng \cdot ml^{-1}$, still more preferably 90±40 $ng \cdot ml^{-1}$, yet more preferably 90±35 $ng \cdot ml^{-1}$, even more preferably 90±30 $ng \cdot ml^{-1}$, most preferably 90±25 $ng \cdot ml^{-1}$, and in particular 90±20 $ng \cdot ml^{-1}$. In still another preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 150±128 $ng \cdot ml^{-1}$, more preferably 150±112 $ng \cdot ml^{-1}$, still more preferably 150±96 $ng \cdot ml^{-1}$, yet more preferably 150±80 $ng \cdot ml^{-1}$, even more preferably 150±64 $ng \cdot ml^{-1}$, most preferably 150±48 $ng \cdot ml^{-1}$, and in particular 150±32 $ng \cdot ml^{-1}$. In yet another preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 300±256 $ng \cdot ml^{-1}$, more preferably 300±224 $ng \cdot ml^{-1}$, still more preferably 300±192 $ng \cdot ml^{-1}$, yet more preferably 300±160 $ng \cdot ml^{-1}$, even more preferably 300±128 $ng \cdot ml^{-1}$, most preferably 300±96 $ng \cdot ml^{-1}$, and in particular 300±64 $ng \cdot ml^{-1}$. In a further preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 600±512 $ng \cdot ml^{-1}$, more preferably 600±448 $ng \cdot ml^{-1}$, still more preferably 600±384 $ng \cdot ml^{-1}$, yet more preferably 600±320 $ng \cdot ml^{-1}$, even more preferably 600±256 $ng \cdot ml^{-1}$, most preferably 600±192 $ng \cdot ml^{-1}$, and in particular 600±128 $ng \cdot ml^{-1}$. In still a further preferred embodiment, the pharmaceutical patch according to the invention provides serum concentrations of the pharmacologically active ingredient over a period of at least 6 hours, more preferably at least 12 hours upon repeated application to the human skin, i.e. under steady state conditions taking into account the depot effect of the skin, of 1,000±800 $ng \cdot ml^{-1}$, more preferably 1,000±700 $ng \cdot ml^{-1}$, still more preferably 1,000±600 $ng \cdot ml^{-1}$, yet more preferably 1,000±500 $ng \cdot ml^{-1}$, even more preferably 1,000±400 $ng \cdot ml^{-1}$, most preferably 1,000±300 $ng \cdot ml^{-1}$, and in particular 1,000±200 $ng \cdot ml^{-1}$. In yet a further preferred embodiment, the pharmaceutical patch according to the invention provides serum concentrations of the pharmacologically active ingredient over a period of at least 6

hours, more preferably at least 12 hours upon repeated application to the human skin, i.e. under steady state conditions taking into account the depot effect of the skin, of 10,000±8,000 $ng \cdot ml^{-1}$, more preferably 10,000±7,000 $ng \cdot ml^{-1}$, still more preferably 10,000±6,000 $ng \cdot ml^{-1}$, yet more preferably 10,000±5,000 $ng \cdot ml^{-1}$, even more preferably 10,000±4,000 $ng \cdot ml^{-1}$, most preferably 10,000±3,000 $ng \cdot ml^{-1}$, and in particular 10,000±2,000 $ng \cdot ml^{-1}$.

The pharmaceutical patch according to the invention preferably contains a crystallization inhibitor which inhibits the crystallization of the pharmacologically active ingredient within the adhesive layer and drug layer, respectively. Thus, the crystallization inhibitor is preferably contained in the same layer as the pharmacologically active ingredient. Preferably, the content of the crystallization inhibitor within said layer is within the range of from 1.0 to 20 wt.-%, more preferably 2.5 to 17.5 wt.-%, still more preferably 5.0 to 15 wt.-%, yet more preferably 6.0 to 14 wt.-%, even more preferably 7.0 to 13 wt.-%, most preferably 8.0 to 12 wt.-%, and in particular 9.0 to 11 wt.-%, relative to the total weight of said layer. Preferred crystallization inhibitors include but are not limited to polyvinylpyrrolidones (povidone, polyvidone) (e.g. Kollidon 25), N-vinyl-1-aza-cycloheptan-2-one homopolymers, N-vinylpiperidin-2-one homopolymers, polyethylene glycol, poloxamer (e.g. Lutrol F127), and copovidone (e.g. Kollidon VA64).

Preferably, the molar ratio of the pharmacologically active ingredient to the crystallization inhibitor is within the range of from 10,000 : 1 to 1 : 10,000, more preferably 5,000 : 1 to 1 : 5,000, still more preferably 1,000 : 1 to 1 : 1,000, yet more preferably 500 : 1 to 1 : 500, even more preferably 100 : 1 to 1 : 100, most preferably 50 : 1 to 1 : 50, and in particular 1 : 1.

In a preferred embodiment, the molar ratio of the pharmacologically active ingredient to the crystallization inhibitor is within the range of 1 : 10±7 (i.e. from 1 : 3 to 1 : 17), more preferably 1 : 10±6, still more preferably 1 : 10±5, yet more preferably 1 : 10±4, even more preferably 1 : 10±3, most preferably 1 : 10±2, and in particular 1 : 10±1. In another preferred embodiment, the molar ratio of the pharmacologically active ingredient to the crystallization inhibitor is within the range of 1 : 20±14, more preferably 1 : 20±12, still more preferably 1 : 20±10, yet more preferably 1 : 20±8, even more preferably 1 : 20±6, most preferably 1 : 20±4, and in particular 1 : 20±2. In still another preferred embodiment, the molar ratio of the pharmacologically active ingredient to the crystallization inhibitor is within the range of 1 : 45±37, more preferably 1 : 45±35, still more preferably 1 : 45±33, yet more preferably 1 : 45±31, even more preferably 1 : 45±29, most preferably 1 : 45±27, and in particular 1 : 45±25. In yet another preferred embodiment, the molar ratio of the pharmacologically active ingredient to the crystallization inhibitor is within the range of 1 : 70±42, more preferably 1 : 70±36, still more preferably 1 : 70±30, yet more preferably 1 : 70±24, even more preferably 1 : 70±18, most preferably 1 : 70±12, and in particular 1 : 70±6. In a further preferred embodiment, the molar ratio of the pharmacologically active ingredient to the crystallization inhibitor is within the range of 1 : 90±70, more preferably 1 : 90±60, still more preferably 1 : 90±50, yet more preferably 1 : 90±40, even more preferably 1 : 90±30, most preferably 1 : 90±20, and in particular 1 : 90±10. In still a further preferred embodiment, the molar ratio of the pharmacologically active ingredient to the crystallization inhibitor is within the range of 1 : 110±70, more preferably 1 : 110±60, still more preferably 1 : 110±50, yet more preferably 1 : 110±40, even more preferably 1 : 110±30, most preferably 1 : 110±20, and in particular 1 : 110±10. In yet a further preferred embodiment, the molar ratio of the pharmacologically active ingredient to the crystallization inhibitor is within the range of 1 : 1,000±700, more preferably 1 : 1,000±600, still more preferably 1 : 1,000±500, yet more preferably 1 : 1,000±400, even more preferably 1 : 1,000±300, most preferably 1 : 1,000±200, and in particular 1 : 1,000±100.

The pharmaceutical patch according to the invention preferably contains a permeation component which enhances percutaneous penetration and permeation of the pharmacologically active ingredient through human skin, i.e. one or more percutaneous penetration enhancers. Percutaneous penetration enhancers are known to the skilled person (cf., e.g., Smith et al., Percutaneous Penetration Enhancers, CRC Press, 1995).

Preferably, the layer of the pharmaceutical patch which contains the pharmacologically active ingredient, i.e. the adhesive layer and/or the drug layer, contains at least one percutaneous penetration enhancer.

Preferably, the relative weight ratio of the pharmacologically active ingredient to the permeation component is within the range of from 1,000 : 1 to 1 : 1,000, more preferably 500 : 1 to 1 : 500, still more preferably 100 : 1 to 1 : 100, most preferably 50 : 1 to 1 : 50, and in particular 25 : 1 to 1 : 25.

Preferably, the molar ratio of the pharmacologically active ingredient to the permeation component is within the range of from 10,000 : 1 to 1 : 10,000, more preferably 5,000 : 1 to 1 : 8,000, still more preferably 1,000 : 1 to 1 : 5,000, yet more preferably 500 : 1 to 1 : 1,000, most preferably 200 : 1 to 1 :500, and in particular 100 : 1 to 1 : 100.

In a preferred embodiment, the molar ratio of the pharmacologically active ingredient to the permeation component is within the range of 1 : 10±8 (i.e. from 1 : 2 to 1 : 18), more preferably 1 : 10±6, most preferably 1 : 10±4, and in particular 1 : 10±2. In another preferred embodiment, the molar ratio of the pharmacologically active ingredient to the permeation component is within the range of 1 : 110±70, more preferably 1 : 110±60, still more preferably 1 : 110±50, yet more preferably 1 : 110±40, even more preferably 1 : 110±30, most preferably 1 : 110±20, and in particular 1 : 110±10. In still another preferred embodiment, the molar ratio of the pharmacologically active ingredient to the permeation component is within the range of 10±8 : 1 (i.e. from 2 : 1 to 18 : 1), more preferably 10±6 : 1, most preferably 10±4 : 1, and in particular 10±2 : 1. In yet another preferred embodiment, the molar ratio of the pharmacologically active

ingredient to the permeation component is within the range of 110±70 : 1, more preferably 110±60 : 1, still more preferably 110±50 : 1, yet more preferably 110±40 : 1, even more preferably 110±30 : 1, most preferably 110±20 : 1, and in particular 110±10 : 1.

Preferably, the permeation component is contained in the same layer of the pharmaceutical patch that also contains the pharmacologically active ingredient or at least a portion thereof.

In a preferred embodiment, particularly when the permeation component comprises isopropyl myristate, oleic acid, dodecanol or sorbitan fatty acid ester, preferably sorbitan monostearate (Span 60), the content of the permeation component within said layer is within the range of from 0.1 to 20 wt.-%, more preferably 0.3 to 16 wt.-%, still more preferably 0.5 to 14 wt.-%, yet more preferably 0.6 to 12 wt.-%, even more preferably 0.7 to 10 wt.-%, most preferably 0.8 to 8 wt.-%, and in particular 0.9 to 6 wt.-%, relative to the total weight of said layer.

In another preferred embodiment, particularly when the permeation component comprises a synthetic wax, the content of the permeation component within said layer is within the range of from 0.1 to 20 wt.-%, more preferably 0.5 to 19 wt.-%, still more preferably 1.0 to 18 wt.-%, yet more preferably 1.3 to 17 wt.-%, even more preferably 1.6 to 16 wt.-%, most preferably 1.9 to 15 wt.-%, and in particular 2 to 14 wt.-%, relative to the total weight of said layer.

In a preferred embodiment, the permeation component comprises at least one percutaneous penetration enhancer having a HLB value (hydrophilic-lipophilic balance) within the range of 1.0±0.8 (i.e. from 0.2 to 1.8), more preferably 1.0±0.7, still more preferably 1.0±0.6, yet more preferably 1.0±0.5, even more preferably 1.0±0.4, most preferably 1.0±0.3, and in particular 1.0±0.2. In another preferred embodiment, the permeation component comprises at least one percutaneous penetration enhancer having a HLB value (hydrophilic-lipophilic balance) within the range of 4.0±3.5, more preferably 4.0±3.0, still more preferably 4.0±2.5, yet more preferably 4.0±2.0, even more preferably 4.0±1.5, most preferably 4.0±1.0, and in particular 4.0±0.5. In still another preferred embodiment, the permeation component comprises at least one percutaneous penetration enhancer having a HLB value (hydrophilic-lipophilic balance) within the range of 8±7, more preferably 8±6, still more preferably 8±5, yet more preferably 8±4, even more preferably 8±3, most preferably 8±2, and in particular 8±1. In yet another preferred embodiment, the permeation component comprises at least one percutaneous penetration enhancer having a HLB value (hydrophilic-lipophilic balance) within the range of 12±7, more preferably 12±6, still more preferably 12±5, yet more preferably 12±4, even more preferably 12±3, most preferably 12±2, and in particular 12±1. In a further preferred embodiment, the permeation component comprises at least one percutaneous penetration enhancer having a HLB value (hydrophilic-lipophilic balance) within the range of 16±7, more preferably 16±6, still more preferably 16±5, yet more preferably 16±4, even more preferably 16±3, most preferably 16±2, and in particular 16±1. In still a further preferred embodiment, the permeation component comprises at least one percutaneous penetration enhancer having a HLB value (hydrophilic-lipophilic balance) within the range of 20±7, more preferably 20±6, still more preferably 20±5, yet more preferably 20±4, even more preferably 20±3, most preferably 20±2, and in particular 20±1. In yet a further preferred embodiment, the permeation component comprises at least one percutaneous penetration enhancer having a HLB value (hydrophilic-lipophilic balance) within the range of 24±7, more preferably 24±6, still more preferably 24±5, yet more preferably 24±4, even more preferably 24±3, most preferably 24±2, and in particular 24±1.

Preferred percutaneous penetration enhancers include but are not limited to:

a) sulfoxides such as dimethylsulfoxide (DMSO) and decylmethylsulfoxide;
b) ethers such as diethylene glycol monoethyl ether (transcutol) and diethylene glycol monomethyl ether;
c) surfactants such as sodium laurate, sodium lauryl sulfate, cetyltrimethylammonium bromide, benzalkonium chloride, poloxamers, polysorbates and lecithin;
d) 1-substituted azacycloheptan-2-ones such as 1-n-dodecylcyclazacycloheptan-2-one;
e) alcohols and fatty alcohols ($C_{10}$-$C_{30}$) such as ethanol, propanol, octanol, dodecanol, oleyl alcohol, benzyl alcohol, and the like;
f) polyols, esters of polyols and ethers of polyols such as propylene glycol, ethylene glycol, diethylene glycol, dipropylene glycol, glycerol, butanediol, polyethylene glycol, and polyethylene glycol monolaurate;
g) organic acids such as salicylic acid and salicylates, citric acid and succinic acid;
h) fatty acids ($C_{10}$-$C_{30}$) such as lauric acid, oleic acid and valeric acid; fatty acid esters (including synthetic waxes) such as isopropyl myristate, isopropyl palmitate, methylpropionate, ethyl oleate, and sorbitan fatty acid esters (e.g. sorbitan monostearate (Span 60));
i) amides and other nitrogenous compounds such as urea, dimethylacetamide, dimethylformamide, 2-pyrrolidone, 1-methyl-2-pyrrolidone, ethanolamine, diethanolamine and triethanolamine;
j) terpenes;
k) alkanones;
l) other oligomers or polymers;

and mixtures of any of the foregoing.

Preferably, the permeation component comprises as percutaneous penetration enhancer an alcohol and/or a fatty acid and/or a fatty acid ester.

In a preferred embodiment, the permeation component comprises as percutaneous penetration enhancer isopropyl myristate and/or oleic acid and/or, dodecanol and/or a sorbitan fatty acid ester, e.g. sorbitan monostearate (Span 60).

In a preferred embodiment, the permeation component comprises as percutaneous penetration enhancer an ester of a fatty acid ($C_{10}$-$C_{30}$) with an alcohol ($C_1$-$C_{12}$), wherein the fatty acid as well as the alcohol independently of one another can be saturated or unsaturated, linear or branched, wherein the fatty acid is preferably selected from the group consisting of caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, and docosahexaenoic acid; and wherein the alcohol is preferably a monoalcohol, preferably selected from the group consisting of methanol, ethanol, propanol, isopropanol, butanol, sec-butanol, and tert-butanol. This embodiment is particularly preferred when the adhesive layer comprises a pressure sensitive adhesive derived from styrene block copolymer-based pressure sensitive adhesives and/or acrylate-based pressure sensitive adhesives, preferably acrylic-rubber-hybrid based pressure sensitive adhesives or acrylate vinyl acetate-based pressure sensitive adhesives, wherein most preferably the pressure sensitive adhesive contains a polymer which is derived from a monomer composition comprising monomer units having at least one hydroxyl functional group.

In a preferred embodiment, the permeation component comprises as percutaneous penetration enhancer isopropyl myristate (HLB ~11.5) in a range of from 0.5 to 13.5 wt.-%, more preferably 2 to 8 wt.-% relative to the total weight of the layer said component is contained in.

In a preferred embodiment, the permeation component comprises as percutaneous penetration enhancer a fatty acid ($C_{10}$-$C_{30}$), which can be saturated or unsaturated, linear or branched, and which is preferably selected from the group consisting of caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, and docosahexaenoic acid. This embodiment is particularly preferred when the adhesive layer comprises a pressure sensitive adhesive derived from acrylate-based pressure sensitive adhesives.

In a preferred embodiment, the permeation component comprises as percutaneous penetration enhancer oleic acid (HLB ~1) in a range of from 0.1 to 8.5 wt.-%, more preferably 1 to 5 wt.-% relative to the total weight of the layer said component is contained in.

In a preferred embodiment, the permeation component comprises a fatty alcohol ($C_{10}$-$C_{30}$) as percutaneous penetration enhancer, which can be saturated or unsaturated, linear or branched, and which is preferably selected from the group consisting of decanol, dodecanol, tetradecanol, hexadecanol and octadecanol. This embodiment is particularly preferred when the adhesive layer comprises a pressure sensitive adhesive derived from styrene block copolymer-based pressure sensitive adhesives and/or acrylate-based pressure sensitive adhesives, preferably acrylic-rubber-hybrid based pressure sensitive adhesives.

In a preferred embodiment, the permeation component comprises as percutaneous penetration enhancer dodecanol (HLB ~1) in a range of from 0.1 to 11 wt.-%, more preferably 1.5 to 6 wt.-% relative to the total weight of the layer said component is contained in.

In a preferred embodiment, the permeation component comprises as percutaneous penetration enhancer an ester, preferably a monoester, of a fatty acid ($C_{10}$-$C_{30}$) with sorbitan, wherein the fatty acid can be saturated or unsaturated, linear or branched, and wherein the fatty acid is preferably selected from the group consisting of caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, and docosahexaenoic acid. Preferred representatives include but are not limited to sorbitan monolaurate (Span® 20), sorbitan monopalmitate (Span® 40), sorbitan monostearate (Span® 60), sorbitan tristearate (Span® 65), sorbitan monooleate (Span® 80), and sorbitan trioleate (Span® 85). This embodiment is particularly preferred when the adhesive layer comprises a pressure sensitive adhesive derived from silicone-based pressure sensitive adhesives and/or acrylate-based pressure sensitive adhesives, preferably acrylic-rubber-hybrid based pressure sensitive adhesives or acrylate vinyl acetate-based pressure sensitive adhesives, wherein most preferably the pressure sensitive adhesive contains a polymer which is derived from a monomer composition comprising monomer units having at least one hydroxyl functional group.

In a preferred embodiment, the permeation component comprises as percutaneous penetration enhancer a sorbitan fatty acid ester, e.g. sorbitan monostearate (Span 60) (HLB ~4.7) in a range of from 0.1 to 7 wt.-%, more preferably 0.5 to 4 wt.-% relative to the total weight of the layer said component is contained in.

Preferably, sorbitan fatty acid esters are effective as solubilizing agents.

Preferably, the pharmaceutical patch has an area of at least 5 $cm^2$, more preferably at least 7.5 $cm^2$, still more

preferably at least 10 $cm^2$, and most preferably at least 15 $cm^2$. In a preferred embodiment, the pharmaceutical patch has an area, i.e. total surface area when being applied to the skin, within the range of 200±150 $cm^2$ (i.e. from 50 $cm^2$ to 350 $cm^2$), more preferably 200±125 $cm^2$, still more preferably 200±100 $cm^2$, yet more preferably 200±75 $cm^2$, even more preferably 200±50 $cm^2$, most preferably 200±25 $cm^2$, and in particular 200±10 $cm^2$. In another preferred embodiment, the pharmaceutical patch has an area within the range of 300±150 $cm^2$, more preferably 300±125 $cm^2$, still more preferably 300±100 $cm^2$, yet more preferably 300±75 $cm^2$, even more preferably 300±50 $cm^2$, most preferably 300±25 $cm^2$, and in particular 300±10 $cm^2$. In still another preferred embodiment, the pharmaceutical patch has an area within the range of 400±150 $cm^2$, more preferably 400±125 $cm^2$, still more preferably 400±100 $cm^2$, yet more preferably 400±75 $cm^2$, even more preferably 400±50 $cm^2$, most preferably 400±25 $cm^2$, and in particular 400±10 $cm^2$.

In a preferred embodiment, the pharmaceutical patch has an area, i.e. total surface area when being applied to the skin, within the range of 25±20 $cm^2$, more preferably 25±15 $cm^2$, still more preferably 25±10 $cm^2$. In another preferred embodiment, the pharmaceutical patch has an area, i.e. total surface area when being applied to the skin, within the range of 50±40 $cm^2$, more preferably 50±35 $cm^2$, still more preferably 50±30 $cm^2$, yet more preferably 50±25 $cm^2$, even more preferably 50±20 $cm^2$, most preferably 50±15 $cm^2$, and in particular 50±10 $cm^2$. In still another preferred embodiment, the pharmaceutical patch has an area within the range of 75±40 $cm^2$, more preferably 75±35 $cm^2$, still more preferably 75±30 $cm^2$, yet more preferably 75±25 $cm^2$, even more preferably 75±20 $cm^2$, most preferably 75±15 $cm^2$, and in particular 75±10 $cm^2$. In yet another preferred embodiment, the pharmaceutical patch has an area within the range of 100±80 $cm^2$, more preferably 100±60 $cm^2$, still more preferably 100±50 $cm^2$, yet more preferably 100±40 $cm^2$, even more preferably 100±30 $cm^2$, most preferably 100±20 $cm^2$, and in particular 100±10 $cm^2$. In a further preferred embodiment, the pharmaceutical patch has an area within the range of 150±80 $cm^2$, more preferably 150±60 $cm^2$, still more preferably 150±50 $cm^2$, yet more preferably 150±40 $cm^2$, even more preferably 150±30 $cm^2$, most preferably 150±20 $cm^2$, and in particular 150±10 $cm^2$. In still a further preferred embodiment, the pharmaceutical patch has an area within the range of 200±80 $cm^2$, more preferably 200±60 $cm^2$, still more preferably 200±50 $cm^2$, yet more preferably 200±40 $cm^2$, even more preferably 200±30 $cm^2$, most preferably 200±20 $cm^2$, and in particular 200±10 $cm^2$. In yet a further preferred embodiment, the pharmaceutical patch has an area within the range of 250±80 $cm^2$, more preferably 250±60 $cm^2$, still more preferably 250±50 $cm^2$, yet more preferably 250±40 $cm^2$, even more preferably 250±30 $cm^2$, most preferably 250±20 $cm^2$, and in particular 250±10 $cm^2$.

Preferably, the area weight of the pharmaceutical patch is within the range of from 1 to 150,000 $g \cdot m^{-2}$, more preferably 10 to 130,000 $g.m^{-2}$ or 3 to 100,000 $g \cdot m^{-2}$, still more preferably 20 to 110,000 $g.m^{-2}$ or 5 to 50,000 $g \cdot m^{-2}$, yet more preferably 40 to 90,000 $g.m^{-2}$ or 7 to 10,000 $g \cdot m^{-2}$, even more preferably 60 to 70,000 $g.m^{-2}$ or 8 to 1,000 $g \cdot m^{-2}$, most preferably 80 to 60,000 $g \cdot m^{-2}$ or 9 to 750 $g \cdot m^{-2}$, and in particular 100 to 50,000 $g \cdot m^{-2}$ or 10 to 500 $g \cdot m\text{-}^{2}$.

In a preferred embodiment, the area weight of the pharmaceutical patch is within the range of from 10 to 300 $g \cdot m^{-2}$, more preferably 13 to 260 $g \cdot m^{-2}$, still more preferably 15 to 230 $g \cdot m^{-2}$, yet more preferably 17 to 200 $g \cdot m^{-2}$, most preferably 19 to 185 $g \cdot m^{-2}$, and in particular 20 to 170 $g \cdot m^{-2}$.

The pharmaceutical patch according to the invention comprises a surface layer.

The term "surface layer" as used herein refers to any layer that represents the surface layer after the application of the pharmaceutical patch. This definition includes permanent backing layers commonly used for pharmaceutical patches as well as thin non-removable films that are typically used in thin flexible patches.

In a preferred embodiment, the surface layer comprises one or more polymers selected from the group consisting of polyurethanes, polyester elastomers, polyether block amides, polyacrylates, ethylene vinyl acetates, ethylene acrylate copolymers, ionomer resins, polyvinyl chloride, polyvinylidene chloride, polyesters and polyolefins, such as polyethylene; polyolefins, in particular polyethylene, polyesters, ethylene vinylacetate copolymers and polyurethanes are particularly preferred.

The surface layer may be a laminate, preferably comprising a polymer film, such as a polyester film, and aluminum foil and/or heat seal layer. In a preferred embodiment, the surface layer consists of a polyester film. Preferably, the surface layer is not coated.

The thickness of the surface layer is not particularly limited. Preferably, the surface layer has a thickness within the range of from 0.1 to 5,000 μm. In a preferred embodiment, the surface layer has a thickness within the range of from 0.5 to 1,000 μm, more preferably from 1 to 750 μm, still more preferably from 5 to 500 μm, most preferably from 10 to 250 μm, and in particular from 20 to 150 μm or from 40 to 100 μm.

In a preferred embodiment, the surface layer has a thickness within the range of 25±20 μm (i.e. from 5 μm to 45 μm), more preferably 25±15 μm, still more preferably 25±10 μm, and yet more preferably 25±5 μm.

The pharmaceutical patch according to the invention comprises a removable protective layer (release liner). Preferably, the removable protective layer comprises a polymer film and a silicone coating or fluoropolymer coating. Preferably, the polymer film is a polyolefin, in particular polyethylene or polypropylene film or polyester, in particular polyethylene terephthalate film. Preferably, the removable protective layer is coated on only one side.

In a preferred embodiment, the removable protective layer is a silicone coated polyester film, such as a silicone coated polyethylene terephthalate film.

In another preferred embodiment, the removable protective layer is a fluoropolymer coated polyester film, such as a fluoropolymer coated polyethylene terephthalate film.

The thickness of the removable protective layer is not particularly limited. Preferably, the removable protective layer has a thickness within the range of from 0.1 to 500 μm. In a preferred embodiment, the removable protective layer has a thickness within the range of from 0.5 to 400 μm, more preferably from 1 to 300 μm, still more preferably from 5 to 250 μm, most preferably from 10 to 200 μm, and in particular from 20 to 150 μm or from 40 to 100 μm.

In a preferred embodiment, the removable protective layer has a thickness within the range of 75±70 μm (i.e. from 5 μm to 145 μm), more preferably 75±60 μm, still more preferably 75±50 μm, yet more preferably 75±40 μm, even more preferably 75±30 μm, most preferably 75±20 μm, and in particular 75±10 μm. In another preferred embodiment, the removable protective layer has a thickness within the range of 100±70 μm, more preferably 100±60 μm, still more preferably 100±50 μm, yet more preferably 100±40 μm, even more preferably 100±30 μm, most preferably 100±20 μm, and in particular 100±10 μm.

The pharmaceutical patch according to the invention comprises an adhesive layer. The adhesive layer comprises at least a portion of the total amount of the pharmacologically active ingredient that is contained in the pharmaceutical patch.

Preferably, the adhesive layer comprises at least 10 wt.-%, more preferably at least 25 wt.-%, still more preferably at least 50 wt.-%, yet more preferably at least 75 wt.-%, even more preferably at least 90 wt.-%, most preferably at least 95 wt.-%, and in particular at least 97 wt.-% of the total amount of the pharmacologically active ingredient that is contained in the pharmaceutical patch.

In another preferred embodiment, the pharmaceutical patch comprises an additional drug layer. According to this embodiment, both the drug layer and the adhesive layer comprise at least a portion of the pharmacologically active compound.

Preferably, the adhesive layer comprises a polymer that forms a matrix in which the pharmacologically active ingredient is dispersed (drug-in-adhesive).

Preferably, the adhesive layer comprises a pressure sensitive adhesive selected from the group consisting of silicone-based pressure sensitive adhesives, acrylate-based pressure sensitive adhesives, acrylate vinyl acetate-based pressure sensitive adhesives, polyisobutylene-based pressure sensitive adhesives, styrene block copolymer-based pressure sensitive adhesives, ethylene vinyl acetate-based pressure sensitive adhesives, polyurethane-based pressure sensitive adhesives and rubber-based pressure sensitive adhesives, preferably acrylic-rubber-hybrid based pressure sensitive adhesives.

The thickness of the adhesive layer is not particularly limited and may depend upon a number of factors such as function within the patch (e.g. drug-in-adhesive), content of pharmacologically active ingredient and excipients, prescribed duration of application of pharmaceutical patch on the skin, and the like. Preferably, the adhesive layer has a thickness within the range of from 1.0 to 1,000 μm.

In a preferred embodiment, the adhesive layer has a thickness within the range of from 50±35 μm (i.e. from 15 μm to 85 μm), more preferably 50±30 μm, still more preferably 50±25 μm, yet more preferably 50±20 μm, even more preferably 50±15 μm, most preferably 50±10 μm, and in particular 50±5 μm. In another preferred embodiment, the adhesive layer has a thickness within the range of from 75±70 μm, more preferably 75±60 μm, still more preferably 75±50 μm, yet more preferably 75±40 μm, even more preferably 75±30 μm, most preferably 75±20 μm, and in particular 75±10 μm. In still another preferred embodiment, the adhesive layer has a thickness within the range of from 100±70 μm, more preferably 100±60 μm, still more preferably 100±50 μm, yet more preferably 100±40 μm, even more preferably 100±30 μm, most preferably 100±20 μm, and in particular 100±10 μm. In yet another preferred embodiment, the adhesive layer has a thickness within the range of from 200±175 μm, more preferably 200±150 μm, still more preferably 200±125 μm, yet more preferably 200±100 μm, even more preferably 200±75 μm, most preferably 200±50 μm, and in particular 200±25 μm. In a further preferred embodiment, the adhesive layer has a thickness within the range of from 300±175 μm, more preferably 300±150 μm, still more preferably 300±125 μm, yet more preferably 300±100 μm, even more preferably 300±75 μm, most preferably 300±50 μm, and in particular 300±25 μm. In still a further preferred embodiment, the adhesive layer has a thickness within the range of from 400±175 μm, more preferably 400±150 μm, still more preferably 400±125 μm, yet more preferably 400±100 μm, even more preferably 400±75 μm, most preferably 400±50 μm, and in particular 400±25 μm. In yet a further preferred embodiment, the adhesive layer has a thickness within the range of from 500±175 μm, more preferably 500±150 μm, still more preferably 500±125 μm, yet more preferably 500±100 μm, even more preferably 500±75 μm, most preferably 500±50 μm, and in particular 500±25 μm.

Preferably, the area weight of the adhesive layer is within the range of from 1 to 150,000 $g \cdot m^{-2}$, more preferably 10 to 130,000 $g.m^{-2}$ or 3 to 100,000 $g \cdot m^{-2}$, still more preferably 20 to 110,000 $g.m^{-2}$ or 5 to 50,000 $g \cdot m^{-2}$, yet more preferably 40 to 90,000 $g.m^{-2}$ or 7 to 10,000 $g \cdot m^{-2}$, even more preferably 60 to 70,000 $g.m^{-2}$ or 8 to 1,000 $g \cdot m^{-2}$, most preferably 80 to 60,000 $g.m^{-2}$ or 9 to 750 $g \cdot m^{-2}$, and in particular 100 to 50,000 $g.m^{-2}$ or 10 to 500 $g \cdot m^{-2}$.

In preferred embodiments a comparatively low area weight may positively influence the shelf-life of the pharmaceutical patch.

The ratio of the thickness of the surface layer to the thickness of the adhesive layer is not particularly limited. In a preferred embodiment, the thickness of the surface layer is greater than the thickness of the adhesive layer. In another preferred embodiment, the thickness of the adhesive layer is greater than the thickness of the surface layer.

In a preferred embodiment, the adhesive layer provides a peel strength (180°, 20 min) of 25±15 N/25 mm, more preferably 25±13 N/25 mm, still more preferably 25±11 N/25 mm, yet more preferably 25±9 N/25 mm, most preferably 25±7 N/25 mm, and in particular 25±5 N/25 mm. In another preferred embodiment, the adhesive layer provides a peel strength of 15±12 N/25 mm, more preferably 15±10 N/25 mm, still more preferably 15±8 N/25 mm, yet more preferably 15±6 N/25 mm, most preferably 15±5 N/25 mm, and in particular 15±4 N/25 mm. In still another preferred embodiment, the adhesive layer provides a peel strength of 5.5±5.0 N/25 mm, more preferably 5.5±4.5 N/25 mm, still more preferably 5.5±4.0 N/25 mm, yet more preferably 5.5±3.5 N/25 mm, even more preferably 5.5±3.0 N/25 mm, most preferably 5.5±2.5 N/25 mm, and in particular 5.5±2.0 N/25 mm. In yet another preferred embodiment, the adhesive layer provides a peel strength of 2.0±1.8 N/25 mm, more preferably 2.0±1.6 N/25 mm, still more preferably 2.0±1.4 N/25 mm, yet more preferably 2.0±1.2 N/25 mm, even more preferably 2.0±1.0 N/25 mm, most preferably 2.0±0.8 N/25 mm, and in particular 2.0±0.6 N/25 mm.

Preferably, the pressure sensitive adhesive has a tack value (loop) in the range of from 1 to 25 N/25 mm. In a preferred embodiment, the pressure sensitive adhesive has a tack value (loop) of 20±15 N/25 mm, more preferably 20±13 N/25 mm, still more preferably 20±11 N/25 mm, yet more preferably 20±9 N/25 mm, most preferably 20±7 N/25 mm, and in particular 20±5 N/25 mm. In another preferred embodiment, the pressure sensitive adhesive has a tack value (loop) of 13±10 N/25 mm, more preferably 13±8 N/25 mm, still more preferably 13±6 N/25 mm, yet more preferably 13±5 N/25 mm, most preferably 13±4 N/25 mm, and in particular 13±3 N/25 mm. In still another preferred embodiment, the pressure sensitive adhesive has a tack value (loop) of 1±0.8 N/25 mm, more preferably 1±0.7 N/25 mm and most preferably 1±0.6 N/25 mm.

In a preferred embodiment, the pressure sensitive adhesive is a silicone-based pressure sensitive adhesive. Preferably, said silicone-based pressure sensitive adhesive forms a matrix in which the pharmacologically active ingredient is embedded. Silicone-based pressure sensitive adhesives are commercially available, e.g. under the trademarks BIO-PSA 7-4601, BIO-PSA 7-4602, BIO-PSA 7-4501, BIO-PSA 7-4502, BIO PSA 7-4503, BIO-PSA 7-4401 and BIO-PSA 7-4402, BIO-PSA 7-4301, BIO-PSA 7-4302, BIO-PSA 7-4201, BIO-PSA 7-4202, BIO-PSA 7-4101, BIO-PSA 7-4102, by Dow Corning Corporation. The silicone-based pressure sensitive adhesive preferably contains a solvent such as ethyl acetate, heptane or toluene and has a solids content of approx. 55-65 wt.-% solids before being dried during the preparation of the patch. The solvents are typically removed during the manufacture of the pharmaceutical patch, though residual traces of solvent may be analytically detectable. Especially preferred silicone based adhesives are commercially available by Dow Corning Corporation under the trademarks BIO PSA 7-4301 (solvent: heptane), BIO PSA 7-4501 (solvent: heptane); BIO PSA 7-4502 (solvent: ethyl acetate); and BIO PSA 7-4503 (solvent: toluene).

In a preferred embodiment, the pressure sensitive adhesive is a silicone-based pressure sensitive adhesive which is supplied in heptane or ethyl acetate. In a preferred embodiment, the pressure sensitive adhesive is BIO PSA 7-4502 or BIO PSA 7-4301.

Preferably, the silicone polymers contained in the silicone-based pressure sensitive adhesives are produced through a condensation reaction of a silanol endblocked polydimethylsiloxane (PDMS) with a silicate resin.

Preferably, the silicone-based pressure sensitive adhesive provides a peel adhesion, preferably measured in accordance with Dow Corning Corp. corporate test method 0964A, of 300±200 $g \cdot cm^{-1}$ (i.e. from 100 $g \cdot cm^{-1}$ to 500 $g \cdot cm^{-1}$), more preferably 300±100 $g \cdot cm^{-1}$, still more preferably 300±50 $g \cdot cm^{-1}$; or 400±200 $g \cdot cm^{-1}$, more preferably 400±100 $g \cdot cm^{-1}$, still more preferably 400±50 $g \cdot cm^{-1}$; or 500±200 $g \cdot cm^{-1}$, more preferably 500±100 $g \cdot cm^{-1}$, still more preferably 500±50 $g \cdot cm^{-1}$; or 600±200 $g \cdot cm^{-1}$, more preferably 600±100 $g \cdot cm^{-1}$, still more preferably 600±50 $g \cdot cm^{-1}$; or 700±200 $g \cdot cm^{-1}$, more preferably 700±100 $g \cdot cm^{-1}$, still more preferably 700±50 $g \cdot cm^{-1}$; or 800±200 $g \cdot cm^{-1}$, more preferably 800±100 $g \cdot cm^{-1}$, still more preferably 800±50 $g \cdot cm^{-1}$; or 900±200 $g \cdot cm^{-1}$, more preferably 900±100 $g \cdot cm^{-1}$, still more preferably 900±50 $g \cdot cm^{-1}$.

In a preferred embodiment, particularly when the pressure sensitive adhesive is a silicone-based pressure sensitive adhesive, the removable protective layer comprises a fluoropolymer coated polyester film.

In preferred embodiments silicone-based pressure sensitive adhesives may stabilize the pharmacologically active ingredient with respect to the formation of undesired degradation products. Formation of said degradation products may be suppressed especially when the concentration of the pharmacologically active ingredient in the adhesive layer that comprises the silicone-based pressure sensitive adhesive is comparatively low.

According to this embodiment, the adhesive layer contains the pharmacologically active ingredient and preferably the silicone-based pressure sensitive adhesive and optional auxiliary substances (excipients) such as one or more percutaneous penetration enhancers, antioxidants, and the like.

According to this embodiment, the concentration of the pharmacologically active ingredient in the adhesive layer that comprises the silicone-based pressure sensitive adhesive is preferably at most 20 wt.-%, more preferably at

most 19 wt.-%, still more preferably at most 18 wt.-%, yet more preferably at most 17 wt.-%, most preferably at most 16 wt.-%, and in particular at most 15 wt.-%, relative to the total weight of the adhesive layer (total per dry unit).

According to this embodiment, the concentration of the pharmacologically active ingredient in the adhesive layer that comprises the silicone-based pressure sensitive adhesive is preferably at least 1.0 wt.-%, more preferably at least 2.0 wt.-%, still more preferably at least 3.0 wt.-%, yet more preferably at least 3.5 wt.-%, even more preferably at least 4.0 wt.-%, most preferably at least 4.5 wt.-%, and in particular at least 5 wt.-%, relative to the total weight of the adhesive layer (total per dry unit).

Preferred compositions of adhesive layers that comprise silicone-based pressure sensitive adhesives are summarized as embodiments $A^1$ to $A^{12}$ in the table here below (all values as percentages relative to the total weight of the adhesive layer, total per dry unit):

| [wt.%] | $A^1$ | $A^2$ | $A^3$ | $A^4$ | $A^5$ | $A^6$ |
|---|---|---|---|---|---|---|
| pharmacologically active ingredient (free base) | 1.0-20.0 | 1.5-19.0 | 2.0-18.0 | 2.5-17.0 | 3.0-16.0 | 3.5-15.0 |
| silicone-based pressure sensitive adhesive | 40.0-99.0 | 42.0-98.5 | 48.0-98.0 | 55.0-96.5 | 61.0-95.5 | 63.0-94.5 |
| percutaneous penetration enhancer(s) | 0-45.0 | 0-40.0 | 0-35.0 | 0-30.0 | 0.4-25.0 | 0.6-20.0 |
| other excipients | 0-18.0 | 0-16.5 | 0-15.0 | 0-13.50 | 0-12.0 | 0-10.5 |
| | | | | | | |
| | $A^7$ | $A^8$ | $A^9$ | $A^{10}$ | $A^{11}$ | $A^{12}$ |
| pharmacologically active ingredient | 4.0-14.0 | 4.5-13.0 | 5.0-12.0 | 5.5-13.0 | 6.0-14.0 | 6.5-15.0 |
| (free base) | | | | | | |
| silicone-based pressure sensitive adhesive | 65.0-93.0 | 67.0-90.0 | 70.0-88.5 | 72.0-87.0 | 73.0-85.5 | 74.0-84.0 |
| percutaneous penetration enhancer(s) | 0.8-17.5 | 1.0-15.0 | 1.2-14.0 | 1.3-13.0 | 1.4-12.0 | 1.5-11.0 |
| other excipients | 0.5-9.0 | 0.5-7.5 | 0.5-6.0 | 0.5-4.5 | 0.5-3.0 | 0.5-1.5 |

According to this embodiment, the adhesive layer comprises the silicone-based pressure sensitive adhesive preferably in combination with a permeation component, preferably comprising an alcohol and/or a fatty acid and/or a fatty acid ester.

According to this embodiment, the adhesive layer comprises the silicone-based pressure sensitive adhesive preferably in combination with a permeation component comprising one or more of isopropyl myristate and/or oleic acid and/or dodecanol and/or a sorbitan fatty acid ester, preferably sorbitan monostearate (Span 60).

In another preferred embodiment, the pressure sensitive adhesive is a styrene block copolymer-based pressure sensitive adhesive. Preferably, said styrene block copolymer-based pressure sensitive adhesive forms a matrix in which the pharmacologically active ingredient is embedded (drug-in-adhesive). The styrene block copolymer-based pressure sensitive adhesive preferably contains a solvent such as toluene or heptane. These solvents are typically removed during the manufacture of the pharmaceutical patch, though residual traces of solvent may be analytically detectable. Especially preferred styrene block copolymer-based pressure sensitive adhesives are commercially available, e.g. under the trademark DuroTAK®, Duro-Tak® 87-6911 (solvent: toluene, heptane), Duro-Tak® 34-4230, Duro-Tak® 9866, Duro-Tak® 4206 and Duro-Tak® 9684.

Examples for styrene block copolymer-based pressure sensitive adhesives include, but are not limited to styrene-butadiene-styrene block copolymers, styrene-ethylene/butylene-styrene block copolymers, styrene-ethylene/propylene block copolymers and styrene-isoprene-styrene block copolymers.

In a preferred embodiment, the styrene block copolymer-based pressure sensitive adhesive is a styrene-butadiene-styrene block copolymer which is supplied in toluene and/or n-heptane.

According to this embodiment, the concentration of the pharmacologically active ingredient in the adhesive layer that comprises the styrene block copolymer-based pressure sensitive adhesive is preferably at most 20 wt.-%, more preferably at most 19 wt.-%, still more preferably at most 18 wt.-%, yet more preferably at most 17 wt.-%, most preferably at most 16 wt.-%, and in particular at most 15 wt.-%, relative to the total weight of the adhesive layer (total

per dry unit).

According to this embodiment, the concentration of the pharmacologically active ingredient in the adhesive layer that comprises the styrene block copolymer-based pressure sensitive adhesive is preferably at least 1.0 wt.-%, more preferably at least 2.0 wt.-%, still more preferably at least 3.0 wt.-%, yet more preferably at least 3.5 wt.-%, even more preferably at least 4.0 wt.-%, most preferably at least 4.5 wt.-%, and in particular at least 5 wt.-%, relative to the total weight of the adhesive layer (total per dry unit).

Preferred compositions of adhesive layers that comprise styrene block copolymer-based pressure sensitive adhesives are summarized as embodiments $B^1$ to $B^{12}$ in the table here below (all values as percentages relative to the total weight of the adhesive layer, total per dry unit):

| [wt.%] | $B^1$ | $B^2$ | $B^3$ | $B^4$ | $B^5$ | $B^6$ |
|---|---|---|---|---|---|---|
| pharmacologically active ingredient (free base) | 1.0-20.0 | 1.5-19.0 | 2.0-18.0 | 2.5-17.0 | 3.0-16.0 | 3.5-15.0 |
| styrene block copolymer-based pressure sensitive adhesive | 40.0-99.0 | 42.0-98.5 | 48.0-98.0 | 55.0-96.5 | 61.0-95.5 | 63.0-94.5 |
| percutaneous penetration enhancer(s) | 0-45.0 | 0-40.0 | 0-35.0 | 0-30.0 | 0.4-25.0 | 0.6-20.0 |
| other excipients | 0-18.0 | 0-16.5 | 0-15.0 | 0-13.50 | 0-12.0 | 0-10.5 |
| | | | | | | |
| | $B^7$ | $B^8$ | $B^9$ | $B^{10}$ | $B^{11}$ | $B^{12}$ |
| pharmacologically active ingredient (free base) | 4.0-14.0 | 4.5-13.0 | 5.0-12.0 | 5.5-13.0 | 6.0-14.0 | 6.5-15.0 |
| styrene block copolymer-based pressure sensitive adhesive | 65.0-93.0 | 67.0-90.0 | 70.0-88.5 | 72.0-87.0 | 73.0-85.5 | 74.0-84.0 |
| percutaneous penetration enhancer(s) | 0.8-17.5 | 1.0-15.0 | 1.2-14.0 | 1.3-13.0 | 1.4-12.0 | 1.5-11.0 |
| other excipients | 0.5-9.0 | 0.5-7.5 | 0.5-6.0 | 0.5-4.5 | 0.5-3.0 | 0.5-1.5 |

According to this embodiment, the adhesive layer contains the pharmacologically active ingredient and preferably the styrene block copolymer-based pressure sensitive adhesive and optional auxiliary substances (excipients) such as one or more percutaneous penetration enhancers, a crystallization inhibitor, antioxidants, and the like.

According to this embodiment, the adhesive layer comprises the styrene block copolymer-based pressure sensitive adhesive preferably in combination with a permeation component, preferably comprising an alcohol and/or a fatty acid and/or a fatty acid ester.

According to this embodiment, the adhesive layer comprises the styrene block copolymer-based pressure sensitive adhesive preferably in combination with a permeation component comprising one or more of isopropyl myristate and/or oleic acid and/or dodecanol and/or a sorbitan fatty acid ester, preferably sorbitan monostearate (Span 60).

In another preferred embodiment, the pressure sensitive adhesive is an acrylate-based pressure sensitive adhesive. Preferably, said acrylate-based pressure sensitive adhesive forms a matrix in which the pharmacologically active ingredient is embedded (drug-in-adhesive). The acrylate-based pressure sensitive adhesive preferably contains a solvent such as ethylacetate, heptane, n-hexane, toluene and isopropanol. These solvents are typically removed during the manufacture of the pharmaceutical patch, though residual traces of solvent may be analytically detectable. Especially preferred acrylate-based pressure sensitive adhesives are commercially available, e.g. under the trademark DuroTAK®, e.g. Duro-Tak® 387-2052 and especially the 87 series, e.g. Duro-Tak® 87-2287; Duro-Tak® 87-4098; Duro-Tak® 87-4287 (solvent: ethylacetate); Duro-Tak® 87-502A (solvent: ethylacetate, heptane and n-hexane); Duro-Tak® 87-2677 (solvent: ethylacetate, isopropanol, heptane and toluene); Duro-Tak® 87-900A (solvent: ethylacetate) and Duro-Tak® 87-9301.

The acrylate-based pressure sensitive adhesive may contain one or more acrylate homopolymers or one or more acrylate copolymers or graft acrylate copolymers or mixtures thereof.

For the purpose of specification, "(meth)acryl" shall refer to both, methacryl as well as acryl.

In a preferred embodiment, the adhesive layer comprises an acrylate copolymer comprising monomer units originating from monomers A which are selected from $C_{1-18}$-alkyl (meth)acrylates and monomers B which are copolymerizable with monomers A. Thus, the acrylate copolymer is derived from at least one monomer of the type of monomers A and at least one monomer of the type of monomers B.

In a preferred embodiment, the acrylate copolymer is derived from two different monomers (bipolymer), three different monomers (terpolymer) or four different monomers (quaterpolymer). Terpolymers are particularly preferred.

Preferred monomers A are selected from the group consisting of methyl (meth)acrylate, ethyl (meth)acrylate, propyl (meth)acrylate, butyl (meth)acrylate, pentyl (meth)acrylate, hexyl (meth)acrylate, cyclohexyl (meth)acrylate, octyl (meth)acrylate, isobornyl (meth)acrylate, and mixtures thereof. 2-Ethylhexyl (meth)acrylate is a preferred representative of an octyl (meth)acrylate.

Preferred monomers B are selected from the group consisting of 2-hydroxyethyl (meth)acrylate, glyceryl mono(meth)acrylate, glycidyl (meth)acrylate, acrylamide, N,N-diethyl(meth)acrylamide, 2-ethoxyethyl (meth)acrylate, 2-ethoxyethoxyethyl (meth)acrylate, tetrahydrofuryl (meth)acrylate, vinyl acetate, N-vinyl pyrrolidone and mixtures thereof.

In a preferred embodiment, the acrylate copolymer is derived from a monomer composition comprising monomer units having at least one hydroxyl functional group, preferably selected from 2-hydroxyethyl (meth)acrylate and glyceryl mono(meth)acrylate.

In a particularly preferred embodiment, the acrylate copolymer is derived from a monomer composition comprising vinyl acetate, 2-ethylhexyl acrylate and 2-hydroxyethyl acrylate (terpolymer), optionally also comprising glycidyl methacrylate (quaterpolymer).

Preferred embodiments $C^1$ to $C^8$ of acrylate copolymers that are preferably contained in the adhesive layer are summarized in the table here below:

| [wt.-%] | $C^1$ | $C^2$ | $C^3$ | $C^4$ | $C^5$ | $C^6$ | $C^7$ | $C^8$ |
|---|---|---|---|---|---|---|---|---|
| vinyl acetate | 5-55 | 10-50 | 15-45 | 20-40 | 20-40 | 20-40 | 20-40 | 20-40 |
| 2-ethylhexyl acrylate | 45-95 | 50-90 | 55-85 | 60-80 | 55-75 | 55-75 | 55-75 | 55-75 |
| 2-hydroxyethyl acrylate | 0-10 | 0-9.0 | 0-8.0 | 0-7.0 | 1.0-9.0 | 2.0-8.0 | 3.0-7.0 | 4.0-6.0 |
| glycidyl methacrylate | 0-5.0 | 0-4.0 | 0-3.0 | 0-2.0 | - | - | - | - |

In a preferred embodiment, the acrylate-based pressure sensitive adhesive is a graft copolymer. According to this embodiment, the side chains of said graft copolymer are hydrocarbon based.

It has been found that different acrylate copolymers, i.e. acrylate copolymers originating from different comonomers and/or different relative amounts of comonomers, have a different influence on its flux rate.

According to this embodiment, the concentration of the pharmacologically active ingredient in the adhesive layer that comprises the acrylate-based pressure sensitive adhesive is preferably at most 20 wt.-%, more preferably at most 19 wt.-%, still more preferably at most 18 wt.-%, yet more preferably at most 17 wt.-%, most preferably at most 16 wt.-%, and in particular at most 15 wt.-%, relative to the total weight of the adhesive layer (total per dry unit).

According to this embodiment, the concentration of the pharmacologically active ingredient in the adhesive layer that comprises the acrylate-based pressure sensitive adhesive is preferably at least 1.0 wt.-%, more preferably at least 2.0 wt.-%, still more preferably at least 3.0 wt.-%, yet more preferably at least 3.5 wt.-%, even more preferably at least 4.0 wt.-%, most preferably at least 4.5 wt.-%, and in particular at least 5 wt.-%, relative to the total weight of the adhesive layer (total per dry unit).

According to this embodiment, the adhesive layer contains the pharmacologically active ingredient and preferably the acrylate-based pressure sensitive adhesive and optional auxiliary substances (excipients) such as one or more percutaneous penetration enhancers, a crystallization inhibitor, antioxidants, and the like.

According to this embodiment, the adhesive layer comprises the acrylate-based pressure sensitive adhesive preferably in combination with a permeation component, preferably comprising an alcohol (e.g. dodecanol) and/or a fatty acid (e.g. oleic acid) and/or a fatty acid ester (e.g isopropyl myristate, sorbitan monostearate).

According to this embodiment, the adhesive layer contains the pharmacologically active ingredient and preferably the acrylate-based pressure sensitive adhesive which is derived from a monomer composition comprising monomer units having at least one hydroxyl functional group, and optional auxiliary substances (excipients) such as one or more percutaneous penetration enhancers, a crystallization inhibitor, antioxidants, and the like.

According to this embodiment, the adhesive layer comprises the acrylate-based pressure sensitive adhesive which is derived from a monomer composition comprising monomer units having at least one hydroxyl functional group preferably in combination with a permeation component, preferably comprising an alcohol (e.g. dodecanol) and/or a fatty acid (e.g. oleic acid) and/or a fatty acid ester (e.g isopropyl myristate, sorbitan monostearate).

In a preferred embodiment, the adhesive layer contains the pharmacologically active ingredient and preferably the pressure sensitive adhesive containing a polymer which is derived from a monomer composition comprising monomer

units having at least one hydroxyl functional group, and optional auxiliary substances (excipients) such as one or more percutaneous penetration enhancers, a crystallization inhibitor, antioxidants, and the like.

According to this embodiment, the adhesive layer comprises the pressure sensitive adhesive containing a polymer which is derived from a monomer composition comprising monomer units having at least one hydroxyl functional group preferably in combination with a permeation component, preferably comprising an alcohol (e.g. dodecanol) and/or a fatty acid (e.g. oleic acid) and/or a fatty acid ester (e.g isopropyl myristate, sorbitan monostearate).

In a preferred embodiment, the acrylate-based pressure sensitive adhesive comprises a polyacrylate vinylacetate copolymer having at least one hydroxyl functional group.

In another preferred embodiment, the acrylate-based pressure sensitive adhesive comprises a polyacrylate graft copolymer having at least one hydroxyl functional group, wherein the grafted side chains are hydrocarbon based.

It has been found that the employment of a pressure sensitive adhesive containing a polymer which is derived from a monomer composition comprising monomer units having at least one hydroxyl functional group provides superior permeation performance of the pharmacologically active ingredient through the skin, particularly in adhesive layers containing acrylate-based pressure sensitive adhesives.

Preferred compositions of adhesive layers that comprise acrylate-based pressure sensitive adhesives are summarized as embodiments $D^1$ to $D^{12}$ in the table here below (all values as percentages relative to the total weight of the adhesive layer, total per dry unit):

| [wt.%] | $D^1$ | $D^2$ | $D^3$ | $D^4$ | $D^5$ | $D^6$ |
|---|---|---|---|---|---|---|
| pharmacologically active ingredient (free base) | 1.0-20.0 | 1.5-19.0 | 2.0-18.0 | 2.5-17.0 | 3.0-16.0 | 3.5-15.0 |
| acrylate-based pressure sensitive adhesive | 40.0-99.0 | 42.0-98.5 | 48.0-98.0 | 55.0-96.5 | 61.0-95.5 | 63.0-94.5 |
| percutaneous penetration enhancer(s) | 0-45.0 | 0-40.0 | 0-35.0 | 0-30.0 | 0.4-25.0 | 0.6-20.0 |
| other excipients | 0-18.0 | 0-16.5 | 0-15.0 | 0-13.50 | 0-12.0 | 0-10.5 |
| | | | | | | |
| | $D^7$ | $D^8$ | $D^9$ | $D^{10}$ | $D^{11}$ | $D^{12}$ |
| pharmacologically active ingredient (free base) | 4.0-14.0 | 4.5-13.0 | 5.0-12.0 | 5.5-13.0 | 6.0-14.0 | 6.5-15.0 |
| acrylate-based pressure sensitive adhesive | 65.0-93.0 | 67.0-90.0 | 70.0-88.5 | 72.0-87.0 | 73.0-85.5 | 74.0-84.0 |
| percutaneous penetration enhancer(s) | 0.8-17.5 | 1.0-15.0 | 1.2-14.0 | 1.3-13.0 | 1.4-12.0 | 1.5-11.0 |
| other excipients | 0.5-9.0 | 0.5-7.5 | 0.5-6.0 | 0.5-4.5 | 0.5-3.0 | 0.5-1.5 |

Preferably, the preferred acrylate copolymers according to any of embodiments $C^1$ to $C^8$ can be contained in any of the preferred compositions of adhesive layers according to any of embodiments $D^1$ to $D^{12}$, i.e.: $C^1D^1$, $C^1D^2$, $C^1D^3$, $C^1D^4$, $C^1D^5$, $C^1D^6$, $C^1D^7$, $C^1D^8$, $C^1D^9$, $C^1D^{10}$, $C^1D^{11}$, and $C^1D^{12}$; $C^2D^1$, $C^2D^2$, $C^2D^3$, $C^2D^4$, $C^2D^5$, $C^2D^6$, $C^2D^7$, $C^2D^8$, $C^2D^9$, $C^2D^{10}$, $C^2D^{11}$, and $C^2D^{12}$; $C^3D^1$, $C^3D^2$, $C^3D^3$, $C^3D^4$, $C^3D^5$, $C^3D^6$, $C^3D^7$, $C^3D^8$, $C^3D^9$ $C^3D^{10}$, $C^3D^{11}$, and $C^3D^{12}$; $C^4D^1$, $C^4D^2$, $C^4D^3$, $C^4D^4$, $C^4D^5$, $C^4D^6$, $C^4D^7$, $C^4D^8$, $C^4D^9$, $C^4D^{10}$, $C^4D^{11}$, and $C^4D^{12}$; $C^5D^1$, $C^5D^2$, $C^5D^3$, $C^5D^4$, $C^5D^5$, $C^5D^6$, $C^5D^7$, $C^5D^8$, $C^5D^9$, $C^5D^{10}$, $C^5D^{11}$, and $C^5D^{12}$; $C^6D^1$, $C^6D^2$, $C^6D^3$, $C^6D^4$, $C^6D^5$, $C^6D^6$, $C^6D^7$, $C^6D^8$, $C^6D^9$, $C^6D^{10}$, $C^6D^{11}$, and $C^6D^{12}$; $C^7D^1$, $C^7D^2$, $C^7D^3$, $C^7D^4$, $C^7D^5$, $C^7D^6$, $C^7D^7$, $C^7D^8$, $C^7D^9$, $C^7D^{10}$, $C^7D^{11}$, and $C^7D^{12}$; $C^8D^1$, $C^8D^2$, $C^8D^3$, $C^8D^4$, $C^8D^5$, $C^8D^6$, $C^8D^7$, $C^8D^8$, $C^8D^9$, $C^BD^{10}$, $C^8D^{11}$, and $C^8D^{12}$.

In a preferred embodiment, the pressure sensitive adhesive contained in the adhesive layer is a silicone-, acrylate- or styrene block copolymer-based pressure sensitive adhesive. For the purpose of the specification, acrylate-based pressure sensitive adhesives also include acrylic-rubber-hybrid adhesives.

In another preferred embodiment, the pressure sensitive adhesive contained in the adhesive layer comprises a copolymer. In still another preferred embodiment, the pressure sensitive adhesive contained in the adhesive layer comprises a graft copolymer.

In yet another preferred embodiment, the pressure sensitive adhesive contained in the adhesive layer comprises a mixture of two or more different pressure sensitive adhesives, e.g. a combination of two different acrylate-based

pressure sensitive adhesives, or a combination of an acrylate-based pressure sensitive adhesive with a silicone-based pressure sensitive adhesive or a combination of an acrylate-based pressure sensitive adhesive with a styrene block copolymer-based pressure sensitive adhesive.

The layer of the pharmaceutical patch that contains the pharmacologically active ingredient or a portion thereof, i.e. the adhesive layer and the drug layer, respectively, may contain other pharmaceutical excipients that are conventionally contained in pharmaceutical patches.

Preferably, the adhesive layer comprises an antioxidant. Suitable antioxidants include but are not limited to alpha-tocopherol, butyl hydroxytoluene or n-propylgalat.

Preferably, the content of the antioxidant is within the range of from 0.01 to 10 wt.-%, more preferably 0.05 to 7.5 wt.-%, still more preferably 0.1 to 2.5 wt.-%, yet more preferably 0.5 to 1.5 wt.-%, even more preferably 0.7 to 1.3 wt.-%, most preferably 0.8 to 1.2 wt.-%, and in particular 0.9 to 1.1 wt.-%, relative to the total weight of the adhesive layer.

In a preferred embodiment, the area of the adhesive layer corresponds to the area of the pharmaceutical patch. In another preferred embodiment, the total area of the adhesive layer can be divided into at least two portions of different composition: an inner area containing the pharmacologically active ingredient and an outer rim surrounding said inner area like a frame, said outer rim preferably not containing pharmacologically active ingredient. The area of said outer rim is not particularly limited but preferably amounts to e.g. about 5% of the total area of the adhesive layer.

In preferred embodiments, the pharmaceutical patch according to the invention exhibits satisfactory storage stability and shelf-life.

Preferably, after storage of the pharmaceutical patch for 3 month at 40°C and 75% relative humidity in a sealed glass container, the degradation of the pharmacologically active ingredient does not exceed 5%, more preferably 4%, still more preferably 3%, yet more preferably 2%, and most preferably 1.5%.

Preferably, after storage of the pharmaceutical patch for 3 month at 5±3°C in a sealed glass container, the degradation of the pharmacologically active ingredient does not exceed 4%, more preferably 3%, still more preferably 2%, yet more preferably 1.5%, most preferably 1%, and in particular 0.75%.

In a preferred embodiment of the pharmaceutical patch according to the invention

- the pharmacologically active ingredient is present in form of the free base, and/or
- the concentration of the pharmacologically active ingredient in the adhesive layer is in the range of from at least 1.0 to 15 wt.-%, preferably at least 2 to 14 wt.-%, most preferably at least 3 to 13 wt.-%, and in particular at least 2 to 12 wt.-%, relative to the total weight of the adhesive layer, and/or
- the pharmaceutical patch upon application to the human skin provides over a period of at least 6 hours release of the pharmacologically active ingredient at a rate of at least 1.0 $ng \cdot cm^{-2} \cdot h^{-1}$, more preferably at least 10 $ng \cdot cm^{-2} \cdot h^{-1}$, still more preferably at least 50 $ng \cdot cm^{-2} \cdot h^{-1}$, yet more preferably at least 100 $ng \cdot cm^{-2} \cdot h^{-1}$, even more preferably at least 500 $ng \cdot cm^{-2} \cdot h^{-1}$, most preferably at least 1,000 $ng \cdot cm^{-2} \cdot h^{-1}$ and in particular at least 10,000 $ng \cdot cm^{-2} \cdot h^{-1}$; and/or
- the pharmaceutical patch provides serum concentrations of the pharmacologically active ingredient over a period of at least 6 hours upon consecutive application of a series of pharmaceutical patches to the human skin, i.e. under steady state conditions taking into account the depot effect of the skin, of 3 to 5,000 $ng \cdot ml^{-1}$, more preferably 4 to 500 $ng \cdot ml^{-1}$ and most preferably 5 to 300 $ng \cdot ml^{-1}$; and/or
- the adhesive layer comprises a polymer that forms a matrix in which the pharmacologically active ingredient is dispersed (drug-in-adhesive) ; and/or
- the adhesive layer comprises an acrylate-based pressure sensitive adhesive, preferably comprising an acrylate copolymer derived from a monomer composition comprising vinyl acetate, 2-ethylhexyl acrylate and 2-hydroxyethyl acrylate (terpolymer), optionally also comprising glycidyl methacrylate; and/or
- the adhesive layer contains one or more percutaneous penetration enhancer(s), preferably an alcohol (e.g. dodecanol) and/or a fatty acid (e.g. oleic acid) and/or a fatty acid ester (e.g. isopropyl myristate or sorbitan monostearate); and/or
- the adhesive layer contains one or more crystallization inhibitors, preferably selected from polyvinylpyrrolidone (e.g. Kollidon 25); and/or
- the adhesive layer comprises an antioxidant in an amount within the range of from 0.01 to 10 wt.-%.

In another preferred embodiment of the pharmaceutical patch according to the invention

- the pharmacologically active ingredient is present in form of the free base, and/or
- the concentration of the pharmacologically active ingredient in the adhesive layer is in the range of from at least 1.0 to 15 wt.-%, preferably at least 2 to 14 wt.-%, most preferably at least 3 to 13 wt.-%, and in particular at least 2 to 12 wt.-%, relative to the total weight of the adhesive layer, and/or
- the adhesive layer comprises a polymer that forms a matrix in which the pharmacologically active ingredient is dispersed (drug-in-adhesive) ; and/or

- the adhesive layer comprises a silicone-based pressure sensitive adhesive; and/or
- the adhesive layer contains one or more percutaneous penetration enhancer(s), preferably an alcohol (e.g. dodecanol) and/or a fatty acid (e.g. oleic acid) and/or a fatty acid ester (e.g. isopropyl myristate or sorbitan monostearate); and/or
- the adhesive layer contains one or more crystallization inhibitors, preferably selected from polyvinylpyrrolidone (e.g. Kollidon 25).

In still another preferred embodiment of the pharmaceutical patch according to the invention

- the pharmacologically active ingredient is present in form of the free base, and/or
- the concentration of the pharmacologically active ingredient in the adhesive layer is in the range of from at least 1.0 to 15 wt.-%, preferably at least 2 to 14 wt.-%, most preferably at least 3 to 13 wt.-%, and in particular at least 2 to 12 wt.-%, relative to the total weight of the adhesive layer, and/or
- the adhesive layer comprises a polymer that forms a matrix in which the pharmacologically active ingredient is dispersed (drug-in-adhesive) ; and/or
- the adhesive layer comprises a styrene block copolymer-based pressure sensitive adhesive and/or
- the adhesive layer contains one or more percutaneous penetration enhancer(s), preferably an alcohol (e.g. dodecanol) and/or a fatty acid (e.g. oleic acid) and/or a fatty acid ester (e.g. isopropyl myristate or sorbitan monostearate); and/or
- the adhesive layer contains one or more crystallization inhibitors, preferably selected from polyvinylpyrrolidone (e.g. Kollidon 25).

In yet another preferred embodiment of the pharmaceutical patch according to the invention

- the pharmacologically active ingredient is present in form of the free base, and/or
- the concentration of the pharmacologically active ingredient in the adhesive layer is in the range of from at least 1.0 to 15 wt.-%, preferably at least 2 to 14 wt.-%, most preferably at least 3 to 13 wt.-%, and in particular at least 2 to 12 wt.-%, relative to the total weight of the adhesive layer, and/or
- the adhesive layer comprises a polymer that forms a matrix in which the pharmacologically active ingredient is dispersed (drug-in-adhesive) ; and/or
- the adhesive layer comprises an acrylic-rubber-hybrid polymer based pressure sensitive adhesive and/or
- the adhesive layer contains one or more percutaneous penetration enhancer(s), preferably an alcohol (e.g. dodecanol) and/or a fatty acid (e.g. oleic acid) and/or a fatty acid ester (e.g. isopropyl myristate or sorbitan monostearate); and/or
- the adhesive layer contains one or more crystallization inhibitors, preferably selected from polyvinylpyrrolidone (e.g. Kollidon 25).

The pharmaceutical patch according to the invention may be prepared by standard techniques for the manufacture of pharmaceutical patches. Such standard techniques are known to the skilled person (cf., e.g., H.A.E. Benson et al., Topical and Transdermal Drug Delivery: Principles and Practice, John Wiley & Sons; 2011; A.K. Banga, Transdermal and Intradermal Delivery of Therapeutic Agents: Application of Physical Technologies, CRC Press Inc; 2011).

Another aspect of the invention relates to a pharmaceutical patch as described above for use in the treatment of pain, preferably moderate to severe acute and chronic pain.

The pharmaceutical patch according to the invention is suitable for use in the treatment of chronic joint pain (osteoarthritis of the hip or knee), low back pain, chronic cancer pain, chronic painful diabetic peripheral neuropathy (DPN), acute dental pain, acute pain after bunionectomy, acute pain after abdominal surgery, acute pain after hip replacement, acute pain after abdominal hysterectomy (visceral pain) and acute pain in patients waiting for joint replacement.

The treatment of pain can either be effected in a systemic therapy or a local therapy. In case of highly localized pain (e.g. bruises or joint pain) a local treatment, i.e. placing the pharmaceutical patch directly on the hurting body part, may be favored providing rapid relief at lower doses of the pharmacologically active ingredient compared to a systemic approach.

In a preferred embodiment, the pharmaceutical patch according to the invention is suitable for use in a systemic treatment of pain. In another preferred embodiment, the pharmaceutical patch according to the invention is suitable for use in a local treatment of pain. Preferably, the pain is moderate, severe, or moderate to severe. In a preferred embodiment, the pain to be treated is neuropathic pain, preferably chronic neuropathic pain such as painful diabetic neuropathy.

For the purpose of specification, neuropathic pain is pain that originates from nerve damage or nerve malfunction. Preferably, the neuropathic pain is selected from acute neuropathic pain and chronic neuropathic pain. Neuropathic pain

may be caused by damage or disease affecting the central or peripheral portions of the nervous system involved in bodily feelings (the somatosensory system). Preferably, the pharmaceutical patch according to the invention is for use in the treatment of chronic neuropathic pain or acute neuropathic pain, peripheral neuropathic pain or central neuropathic pain, mononeuropathic pain or polyneuropathic pain. When the neuropathic pain is chronic, it may be chronic peripheral neuropathic pain or chronic central neuropathic pain, in a preferred embodiment chronic peripheral mononeuropathic pain or chronic central mononeuropathic pain, in another preferred embodiment chronic peripheral polyneuropathic pain or chronic central polyneuropathic pain. When the neuropathic pain is acute, it may be acute peripheral neuropathic pain or acute central neuropathic pain, in a preferred embodiment acute peripheral mononeuropathic pain or acute central mononeuropathic pain, in another preferred embodiment acute peripheral polyneuropathic pain or acute central polyneuropathic pain. The invention also relates to the pharmacologically active ingredient according to the invention a physiologically acceptable salt thereof for use in the treatment of neuropathic pain as described above.

Central neuropathic pain is found in spinal cord injury, multiple sclerosis, and some strokes. Fibromyalgia is potentially a central pain disorder and is responsive to medications that are effective for neuropathic pain. Accordingly, the pharmaceutical patch according to the invention is also suitable for the treatment of fibromyalgia. Aside from diabetic neuropathy and other metabolic conditions, the common causes of painful peripheral neuropathies are herpes zoster infection, HIV-related neuropathies, nutritional deficiencies, toxins, remote manifestations of malignancies, genetic, and immune mediated disorders or physical trauma to a nerve trunk. Neuropathic pain is common in cancer as a direct result of cancer on peripheral nerves (e.g., compression by a tumor), or as a side effect of chemotherapy, radiation injury or surgery.

The pharmaceutical patch according to the invention is also suitable for use in the treatment of nociceptive pain, preferably acute or chronic nociceptive pain. Preferably, the pain is moderate, severe, or moderate to severe.

Nociceptive pain refers to the discomfort that results when a stimulus causes tissue damage to the muscles, bones, skin or internal organs. For the purpose of specification, nociceptive pain is caused by stimulation of peripheral nerve fibers that respond only to stimuli approaching or exceeding harmful intensity (nociceptors), and may be classified according to the mode of noxious stimulation; the most common categories being "thermal" (heat or cold), "mechanical" (crushing, tearing, etc.) and "chemical" (iodine in a cut, chili powder in the eyes). Nociceptive pain may also be divided into "visceral," "deep somatic" and "superficial somatic" pain.

Visceral pain describes a type of nociceptive pain originating in the body's internal organs or their surrounding tissues. This form of pain usually results from the infiltration of harmful cells, as well as the compression or extension of healthy cells. Patients suffering from visceral pain tend to feel generally achy, as this pain tends to not be localized to a specific area. Cancer is a common source of visceral pain.

Somatic pain is nociceptive pain that results from some injury to the body. It's generally localized to the affected area and abates when the body repairs the damage to that area. Deep somatic pain is initiated by stimulation of nociceptors in ligaments, tendons, bones, blood vessels, fasciae and muscles, and is dull, aching, poorly-localized pain. Examples include sprains and broken bones. Superficial pain is initiated by activation of nociceptors in the skin or superficial tissues, and is sharp, well-defined and clearly located.

According to the invention, nociceptive pain is preferably classified chronic if it has occurred for at least 3 months. Preferably, the chronic nociceptive pain is selected from chronic visceral pain, chronic deep somatic pain and chronic superficial somatic pain.

Preferred causes of nociceptive pain according to the invention include broken or fractured bones, bruises, burns, cuts, inflammation (from infection or arthritis), and sprains. Thus, nociceptive pain includes post-operative pain, cancer pain, low back pain, and inflammatory pain.

In preferred embodiments, the pain to be treated is selected from the group consisting of pain being or being associated with panic disorder [episodic paroxysmal anxiety] [F41.0]; dissociative [conversion] disorders [F44]; persistent somatoform pain disorder [F45.4]; pain disorders exclusively related to psychological factors [F45.41]; nonorganic dyspareunia [F52.6]; other enduring personality changes [F62.8]; sadomasochism [F65.5]; elaboration of physical symptoms for psychological reasons [F68.0]; migraine [G43]; other headache syndromes [G44]; trigeminal neuralgia [G50.0]; atypical facial pain [G50.1]; phantom limb syndrome with pain [G54.6]; phantom limb syndrome without pain [G54.7]; acute and chronic pain, not elsewhere classified [G89]; ocular pain [H57.1]; otalgia [H92.0]; angina pectoris, unspecified [I20.9]; other specified disorders of nose and nasal sinuses [J34.8]; other diseases of pharynx [J39.2]; temporomandibular joint disorders [K07.6]; other specified disorders of teeth and supporting structures [K08.8]; other specified diseases of jaws [K10.8]; other and unspecified lesions of oral mucosa [K13.7]; glossodynia [K14.6]; other specified diseases of anus and rectum [K62.8]; pain in joint [M25.5]; shoulder pain [M25.51]; sacrococcygeal disorders, not elsewhere classified [M53.3]; spine pain [M54.]; radiculopathy [M54.1]; cervicalgia [M54.2]; sciatica [M54.3]; low back pain [M54.5]; pain in thoracic spine [M54.6]; other dorsalgia [M54.8]; dorsalgia, unspecified [M54.9]; other shoulder lesions [M75.8]; other soft tissue disorders, not elsewhere classified [M79]; myalgia [M79.1]; neuralgia and neuritis, unspecified [M79.2]; pain in limb [M79.6]; other specified disorders of bone [M89.8]; unspecified renal colic [N23]; other specified disorders of penis [N48.8]; other specified disorders of male genital organs [N50.8]; mastodynia [N64.4]; pain and other conditions

associated with female genital organs and menstrual cycle [N94]; mittelschmerz [N94.0]; other specified conditions associated with female genital organs and menstrual cycle [N94.8]; pain in throat and chest [R07]; pain in throat [R07.0]; chest pain on breathing [R07.1]; precordial pain [R07.2]; other chest pain [R07.3]; chest pain, unspecified [R07.4]; abdominal and pelvic pain [R10]; acute abdomen pain [R10.0]; pain localized to upper abdomen [R10.1]; pelvic and perineal pain [R10.2]; pain localized to other parts of lower abdomen [R10.3]; other and unspecified abdominal pain [R10.4]; flatulence and related conditions [R14]; abdominal rigidity [R19.3]; other and unspecified disturbances of skin sensation [R20.8]; pain associated with micturition [R30]; other and unspecified symptoms and signs involving the urinary system [R39.8]; headache [R51]; pain, not elsewhere classified [R52]; acute pain [R52.0]; chronic intractable pain [R52.1]; other chronic pain [R52.2]; pain, unspecified [R52.9]; other complications of cardiac and vascular prosthetic devices, implants and grafts [T82.8]; other complications of genitourinary prosthetic devices, implants and grafts [T83.8]; other complications of internal orthopaedic prosthetic devices, implants and grafts [T84.8]; other complications of internal prosthetic devices, implants and grafts, not elsewhere classified [T85.8]; wherein the information in brackets refers to the classification according to ICD-10.

Preferably, the pharmaceutical patch is designed for application to the skin for a period of least 1 day, more preferably at least 2 days, most preferably at least 3 days or at least 3.5 days, and in particular 3 days, 3.5 days, 4 days or 7 days. Thus, according to this embodiment, continuous administration of the pharmacologically active ingredient can be achieved by removing a used pharmaceutical patch after the predetermined period has expired and replacing it by a fresh pharmaceutical patch.

The locations of the skin to which the pharmaceutical patch according to the invention is to be applied are not particularly limited. Preferably, the pharmaceutical patch according to the invention is applied to the skin of the breast or the skin of the back or the skin of the inner arm.

In a preferred embodiment, the pharmaceutical patches according to the invention are repeatedly applied to the same location on the skin, i.e. after a first pharmaceutical patch has been used and needs to be replaced by a second pharmaceutical patch in order to maintain the desired pharmacological effect, said second pharmaceutical patch is preferably applied to the same location on the skin to which said first pharmaceutical patch was applied before.

In another preferred embodiment, particularly when the patient has a sensitive skin, the pharmaceutical patches according to the invention are applied to the different locations on the skin, i.e. after a first pharmaceutical patch has been used and needs to be replaced by a second pharmaceutical patch in order to maintain the desired pharmacological effect, said second pharmaceutical patch is preferably applied to a location on the skin differing from the location on the skin to which said first pharmaceutical patch was applied before.

The pharmaceutical patch according to the invention is for administration to the skin of a mammal, preferably of a human (pediatrics or adults).

The following examples further illustrate the invention but are not to be construed as limiting its scope.

## EXAMPLES

In the following, "API" refers to the pharmacologically active ingredient in form of its free base and "API HCl" corresponds to the pharmacologically active ingredient in form of its hydrochloride addition salt.

Furthermore, in the following, all amounts of the pharmacologically active ingredient (i.e. API and API HCl) are given as the respective corresponding amount of the free base.

HPLC method:

Precolumn: Merck Select B 4-4; Column: Merck Select B 125-3, 5 μm, Nr. 30; Eluent A: 20 mM phosphate buffer + 1 mL/L triethylamine, pH 2.5, 68 %; Eluent B: methanol, 32 %; Flow: 0.65 mL/min;
Run time: 6 min; Detection: UV; Wavelength: 215 nm; Injection volume: variable between 1-50 μL; Oven temperature: 40°C; Sample rack temperature: 20°C; $R_t$ (API HCl): 4.17 min; Bandwidth: 0.32 min;
Asymmetry: 1.41; Theoretical plates: 2673.

### Example 1: *Solubility*

The solubility of API and API HCl in different organic solvents and aqueous solutions is tested by transferring defined volumes of the solvents into micro vials. API and API HCl, respectively, are added until saturation. The solutions are centrifuged and the supernatants analyzed by HPLC (Table 1).

**Table 1:** solubility of API and API HCl in different solvents.

| solvent | API [mg/mL] | API HCl [mg/mL] |
|---|---|---|
| water (pH = 5.8) | 0.48 | 298 |
| HEPES (pH = 7.2) | 5.43 | 286 |
| ethylacetate | 232 | 4.46 |
| ethanol | 404 | 33.1 |
| *n*-heptane | 1.27 | - |
| acetone | 332 | 0.92 |

It becomes clear from the above experimental data that Tapentadol in form of its free base (API) is soluble in all analyzed organic solvents except for n-heptane. In the other solvents API contents higher than 230 mg/mL are measured. It appears that the amount of API which is dissolved in the solution increases with increasing polarity of the solvent. In the aqueous solutions, the dissolved amounts of API do not exceed 5.5 mg/mL.

The hydrochloride salt of Tapentadol (API HCl) exhibits excellent solubility in aqueous solutions but is hardly soluble in organic solvents like ethylacetate or acetone and in particular ethanol and n-heptane.

Example 2: *Compatibility with adhesives*

The compatibility of the hydrochloride salt of Tapentadol (API HCl) with different types of adhesives is tested (Table 2).

Adhesives used:

| | |
|---|---|
| Duro-Tak 87-900A: | non-functional acrylic (no hydroxyl, no carboxyl groups), non-curing |
| Duro-Tak 87-4287: | hydroxyl-functionalized polyacrylate-vinylacetate copolymer, non-curing |
| Duro-Tak 87-2677: | carboxylate-functionalized polyacrylate-vinylacetate copolymer, self-curing |
| Duro-Tak 87-6911A: | tackified styrene rubber (styrene-block-butadiene-block-styrene), not functionalized |
| Bio PSA 7-4502: | silicone adhesive. |

**Table 2:** compatibility of API HCl with different adhesives.

| adhesive | solids in solution [%] | API HCl in solution [%] |
|---|---|---|
| DT 87-2677 | 32 | 5 |
| DT 87-4287 | 32 | 5 |
| DT 87-900A | 32 | 5 |
| DT 87-6911A | 32 | 2 |
| Bio PSA 7-4502 | 32 | 2 |
| DT 87-2677 | 32 | 10 |
| DT 87-4287 | 32 | 10 |
| DT 87-6911A | 32 | 5 |
| DT 87-900A | 32 | 10 |
| Bio PSA 7-4502 | 32 | 5 |

The two samples containing the silicone adhesive Bio PSA 7-4502 yield both clear and colorless solutions containing 2 and 5 % of API HCl, respectively. The samples containing the acrylate adhesives show different behaviors depending on the specific polymer type and the functionality. The hydroxyl-functionalized polymer DT 87-4287 gives opaque solutions which turn slightly yellow after two weeks at room temperature. This effect is more pronounced with the carboxylate-functionalized DT 87-2677 although the yellowish solutions are clear. The solutions of the non-functionalized DT 87-900A are clear and colorless. Although its solubilization power was poor in comparison, the styrene-block-butadiene-block-styrene Duro-Tak 87-6911A is beneficial for the chemical stability of Tapentadol as it lacks reactive functional groups.

Example 3: *Skin permeation from donor solution*

Saturated solutions of API and API HCl were prepared in a mixture of ethanol and dodecanol (95 : 5) and ethanol and oleic acid (85 : 15), respectively.

An in-vitro permeation study was performed with the saturated solutions to show permeation of drug through mouse skin. The in-vitro experiments were conducted using a Franz-diffusion cell. The Franz-diffusion cell consists of a donor compartment and an acceptor compartment.

| | |
|---|---|
| Skin: | hairless mouse skin, female, age 6-7 weeks |
| Stem: | Hsd: Athymic Nude-Foxn1$^{nu}$ |
| Dermal tissue: | dorsal and abdominal with all dermal layers |
| Breeder: | Harlan Laboratories GmbH |
| Acceptor medium: | HEPES buffer pH 7.2 |
| Volume acceptor: | 15 mL |
| Volume donor: | 1.0 mL |
| Donor solutions: | API HCl in ethanol/dodecanol (95/5)<br>API HCl in ethanol/oleic acid (85/15)<br>API in ethanol/dodecanol (95/5)<br>API in ethanol/oleic acid (85/15) |
| Permeation area: | ~1.00 cm$^2$ |
| Temperature in donor solution: | 32 ± 0.5°C |
| Sampling times: | 3 h, 6 h, 12 h, 18 h, 24 h |
| Sampling: | automatic sampling system #115 |
| Rinsed volume: | ~7.0 mL |
| Sample volume: | 1.0 mL |
| Replaced volume: | 8.0 mL |
| Standards: | API HCl in acceptor medium<br>c(std A) = 86.12 µg/mL<br>c(std B) = 8.612 µg/mL |
| HPLC method: | see above |
| Injection volume: | variable between 3-15 µL and adjusted dilution factor |

**Table 3:** sample information.

| | API HCl in ethanol/ dodecanol (95 : 5) | | API HCl in ethanol/ oleic acid (85 : 15) | | API in ethanol/ dodecanol (95 : 5) | | API in ethanol/oleic acid (85 : 15) | |
|---|---|---|---|---|---|---|---|---|
| sample weight [mg] | 10.0 | 10.0 | 10.0 | 10.0 | 10.0 | 10.0 | 10.0 | 10.0 |
| replaced volume IS [ml] | 7.1 | 7.1 | 7.1 | 7.1 | 7.1 | 7.1 | 7.1 | 7.1 |
| replaced volume target [ml] | 8.0 | 8.0 | 8.0 | 8.0 | 8.0 | 8.0 | 8.0 | 8.0 |
| API or API HCl content [mg/cm$^2$] | 45.77 | 45.77 | 39.33 | 39.33 | 51.30 | 51.30 | 49.10 | 49.10 |
| diss. volume [ml] | 15.0 | 15.0 | 15.0 | 15.0 | 15.0 | 15.0 | 15.0 | 15.0 |
| permeation area [cm$^2$] | 0.98 | 0.98 | 0.98 | 0.98 | 0.98 | 0.98 | 0.98 | 0.98 |

**Table 4:** concentration of API or API HCl, respectively, in sample solution.

| concentration of API or API HCl in sample solution [μg/ml] | | | | | | | | |
|---|---|---|---|---|---|---|---|---|
| total time (h) | API HCl in ethanol/ dodecanol (95 : 5) | | API HCl in ethanol/oleic acid (85 : 15) | | API in ethanol/ dodecanol (95 : 5) | | API in ethanol/oleic acid (85 : 15) | |
| 3 | 23.856 | 5.137 | 10.859 | 12.409 | 34.914 | 51.610 | 53.604 | 86.606 |
| 6 | 52.893 | 19.220 | 26.807 | 34.414 | 79.955 | 105.327 | 278.914 | 417.58 1 |
| 12 | 203.526 | 66.696 | 94.462 | 109.882 | 351.444 | 469.548 | 326.763 | 316.73 9 |
| 18 | 508.887 | 177.308 | 105.741 | 118.759 | 1128.078 | 1224.529 | 262.813 | 466.28 |
| | | | | | | | | 8 |
| 24 | 585.603 | 281.719 | 100.635 | 107.927 | 997.460 | 1052.063 | 362.673 | 395.53 4 |

**Table 5A:** total permeated amount of API or API HCl, respectively.

| total permeated amount of API or API HCl [μg/cm$^2$] | | | | | | | | |
|---|---|---|---|---|---|---|---|---|
| total dissolution time (h) | API HCl in ethanol/ dodecanol (95 : 5) | | API HCl in ethanol/oleic acid (85 : 15) | | API in ethanol/ dodecanol (95 : 5) | | API in ethanol/oleic acid (85 : 15) | |
| 3 | 365.1 | 78.6 | 166.2 | 189.9 | 534.4 | 789.9 | 820.5 | 1325.6 |
| 6 | 982.4 | 331.4 | 489.0 | 616.7 | 1476.8 | 1986.1 | 4657.4 | 7019.0 |
| 12 | 3671.2 | 1197.3 | 1718.7 | 2021.1 | 6211.5 | 8324.0 | 7410.5 | 8500.8 |
| 18 | 9819.6 | 3373.6 | 2575.7 | 2953.1 | 20644.9 | 23281.6 | 8799.1 | 13084.6 |
| 24 | 14680.7 | 6256.3 | 3263.7 | 3647.7 | 26818.4 | 29513.4 | 12231.6 | 15379.8 |

**Table 5B:** statistical analysis over all values of Table 5A:

| total dissolution time (h) | mean | RSD (%) | min. | max. |
|---|---|---|---|---|
| 3 | 533.8 | 79.6 | 78.6 | 1325.6 |
| 6 | 2194.8 | 109.2 | 331.4 | 7019.0 |
| 12 | 4881.9 | 63.1 | 1197.7 | 8500.8 |
| 18 | 10566.5 | 75.6 | 2575.7 | 23281.6 |
| 24 | 13973.9 | 71.2 | 3263.7 | 29513.4 |

It becomes clear from the above experimental data in Tables 5A and 5B that the free base of Tapentadol passes across the skin barrier much better than the hydrochloride of Tapentadol. Further, it becomes clear from the above data that under the given conditions, dodecanol is more effective as skin permeation enhancer than oleic acid. The data of Table 5A is also shown in Figure 1.

**Table 6A:** percentage of permeated amount of API or API HCl, respectively.

| permeated amount of API or API HCl [%] | | | | | | | | |
|---|---|---|---|---|---|---|---|---|
| total dissolution time (h) | API HCl in ethanol/ dodecanol (95 : 5) | | API HCl in ethanol/oleic acid (85 : 15) | | API in ethanol/ dodecanol (95 : 5) | | API in ethanol/oleic acid (85 : 15) | |
| 3 | 0.8 | 0.2 | 0.4 | 0.5 | 1.0 | 1.5 | 1.7 | 2.7 |
| 6 | 2.1 | 0.7 | 1.2 | 1.6 | 2.9 | 3.9 | 9.5 | 14.3 |

(continued)

| permeated amount of API or API HCl [%] | | | | | | | | |
|---|---|---|---|---|---|---|---|---|
| total dissolution time (h) | API HCl in ethanol/ dodecanol (95 : 5) | | API HCl in ethanol/oleic acid (85 : 15) | | API in ethanol/ dodecanol (95 : 5) | | API in ethanol/oleic acid (85 : 15) | |
| 12 | 8.0 | 2.6 | 4.4 | 5.1 | 12.1 | 16.2 | 15.1 | 17.3 |
| 18 | 21.5 | 7.4 | 6.5 | 7.5 | 40.2 | 45.4 | 17.9 | 26.6 |
| 24 | 32.1 | 13.7 | 8.3 | 9.3 | 52.3 | 57.5 | 24.9 | 31.3 |

**Table 6B:** statistical analysis over all values of Table 6A:

| total dissolution time (h) | mean | RSD (%) | min. | max. |
|---|---|---|---|---|
| 3 | 1.1 | 75.6 | 0.2 | 2.7 |
| 6 | 4.5 | 106.5 | 0.7 | 14.3 |
| 12 | 10.1 | 57.5 | 2.6 | 17.3 |
| 18 | 21.6 | 69.4 | 6.5 | 45.4 |
| 24 | 28.7 | 65.1 | 8.3 | 57.5 |

It again becomes clear from the above experimental data in Tables 6A and 6B that the free base of Tapentadol passes across the skin barrier much better than the hydrochloride of Tapentadol. Further, it becomes clear from the above data that under the given conditions, dodecanol is more effective as skin permeation enhancer than oleic acid. The data of Table 6A is also shown in Figure 2.

**Table 7A:** flux in [$\mu g/cm^2h$].

| flux in [$\mu g/cm^2h$] | | | | | | | | |
|---|---|---|---|---|---|---|---|---|
| time between sampling (h) | API HCl in ethanol/ dodecanol (95 : 5) | | API HCl in ethanol/ oleic acid (85 : 15) | | API in ethanol/ dodecanol (95 : 5) | | API in ethanol/oleic acid (85 : 15) | |
| 3 | 121.7 | 26.2 | 55.4 | 63.3 | 178.1 | 263.3 | 273.5 | 441.9 |
| 3 | 205.8 | 84.3 | 107.6 | 142.2 | 314.1 | 398.7 | 1279.0 | 1897. 8 |
| 6 | 448.1 | 144.3 | 205.0 | 234.1 | 789.1 | 1056.3 | 458.8 | 247.0 |
| 6 | 1024.7 | 362.7 | 142.8 | 155.3 | 2405.6 | 2492.9 | 231.4 | 764.0 |
| 6 | 810.2 | 480.5 | 114.7 | 115.8 | 1028.9 | 1038.6 | 572.1 | 382.5 |

**Table 7B:** statistical analysis over all values of Table 7A:

| time between sampling (h) | mean | RSD (%) | min. | max. |
|---|---|---|---|---|
| 3 | 177.9 | 79.6 | 26.2 | 441.9 |
| 3 | 553.7 | 120.7 | 84.3 | 1897.8 |
| 6 | 447.8 | 71.8 | 144.3 | 1056.3 |
| 6 | 947.4 | 103.1 | 142.8 | 2492.9 |
| 6 | 567.9 | 64.7 | 114.7 | 1038.6 |

Example 4: *Permeation of Tapentadol from adhesives*

Adhesive formulations of API at 5 wt.-% (in dry unit) in different adhesives were prepared in 20 mL vials and

mixed on a jar roller. Ethyl acetate was utilized as solvent for dilution. The target solids content was 35 %. The formulations were used to cast one sheet on a silicone coated polyester or polyethylene film (PET, 75 μm, Loparex Primeliner® FLS release liner). A Gardco Automatic Drawdown machine was used to spread the adhesive at a thickness to target 50-80 g/$m^2$ with a coating thickness of from 0.224 to 0.233 mm. The sheet was dried for 15 minutes at 60°C. It was laminated with a polyester laminate (PETP, 23 μm, 3M Scotchpak® 9754 Surface layer, polyester film with an ethylene vinylacetate copolymer heat seal layer). Some 3.9 $cm^2$ round patches were cut from each sheet and packaged in heat sealed foil pouches. The batch size dry weight amounted to 5 g.

Permeation was tested by application of patches on the skin of mice for 24 h. The experiment was realized as a double determination at 32°C with a permeation area of 0.98 $cm^2$. After 3, 6, 12, 18 and 24 h blood samples were drawn and the API content was determined *via* HPLC.

| | |
|---|---|
| Skin: | hairless mouse skin, female, age 6-7 weeks |
| Stem: | Hsd: Athymic Nude-Foxn1$^{nu}$ |
| Dermal tissue: | dorsal and abdominal with all dermal layers |
| Breeder: | Harlan Laboratories GmbH |
| Acceptor medium: | HEPES buffer pH 7.2 |
| Volume acceptor: | 15 mL |
| Patch size: | 0.98 $cm^2$ |
| Permeation area: | 1.00 $cm^2$ |
| Size of skin: | 3.66 $cm^2$ |
| Size placebo ring: | 3.66 $cm^2$ |
| Temperature in acceptor solution: | 32 ± 0.5°C |
| Sampling times: | 3 h, 6 h, 12 h, 18 h, 24 h |
| Sampling: | automatic sampling system #115 |
| Rinsed volume: | 7.0 mL |
| Sample volume: | 1.0 mL |
| Replaced volume: | 8.0 mL |
| Standards: | API HCl in acceptor medium |
| | c(std A) = 99.60 μg/mL |
| | c(std B) = 98.74 μg/mL |
| HPLC method: | see above |
| Injection volume: | 25.0 μL |
| Adhesives used: | |
| Duro-Tak 87-900A: | non-functional acrylic (no hydroxyl, no carboxyl groups), non-curing |
| Durotak 87-4287: | hydroxyl-functionalized polyacrylate-vinylacetate copolymer, non-curing |
| Durotak 387-2052: | carboxylate-functionalized acrylic. |

**Table 8:** sample information.

| sample no. | content of API in dry unit [%] | content of API [mg/$cm^2$] | coating weight per area [g/$m^2$] | adhesive |
|---|---|---|---|---|
| 1 | 5 | 0.27 | 58.2 | DT 87-900A |
| 2 | 5 | 0.25 | 50.8 | DT 87-4287 |
| 3 | 5 | 0.26 | 53.5 | DT 387-2052 |
| 4 | 5 | 0.38 | 80.3 | DT 87-900A |
| 5 | 5 | 0.31 | 63.3 | DT 87-4287 |
| 6 | 5 | 0.29 | 70.2 | DT 387-2052 |

The results from the permeation test are shown in Figures 3 and 4.

It becomes clear from the Figures 3 and 4 that the skin permeation of Tapentadol is dependent on the different

types of acrylate adhesives. Whereas the permeation from the hydroxyl-modified adhesive Durotak 87-4287 increases until draining of the single samples 2 and 5, in the samples 3 and 6 containing the acidic adhesive Durotak 387-2052 only small quantities of the drug are detected. This could be attributed to the protonation of the amino function of Tapentadol which prevents its skin permeation. The employment of Durotak 87-900A (samples 1 und 4) which lacks functionality results in medium permeation rates. It appears that hydroxyl groups in the polymer matrix are beneficial for the permeation.

Example 5: *Permeation of Tapentadol from adhesives*

Different classes of adhesive polymers, i.e. hydroxyl-modified polyacrylates, hybrid PSA synthesized from polyacrylates and styrene-butadiene, styrene-block-butadiene-block-styrene (SBS) adhesives and silicones; and commonly used percutaneous penetration enhancers (isopropyl myristate, oleic acid, dodecanol and Span 60) are investigated.

The results from the permeation tests are shown in Figures 5 to 12.

**Table 9:** sample information.

| Figure | sample no. | adhesive [%] | | API [%] | isopropyl myristate [%] | oleic acid [%] | dodecanol [%] | Span 60 [%] |
|---|---|---|---|---|---|---|---|---|
| 5 | 1 | DT 87-4287 | 91.50 | 6.50 | - | - | - | 2.00 |
| | 2 | | 90.00 | 6.00 | 4.00 | - | - | - |
| | 3 | | 90.00 | 10.00 | - | - | - | - |
| 6 | 4 | | 93.00 | 5.00 | - | 2.00 | - | - |
| | 5 | | 91.25 | 5.75 | - | - | 3.00 | - |
| | 6 | | 95.00 | 5.00 | - | - | - | - |
| 7 | 7 | DT 87-502A | 91.50 | 6.50 | | | | 2.00 |
| | 8 | | 90.00 | 6.00 | 4.00 | - | - | - |
| | 9 | | 90.00 | 10.00 | - | - | - | - |
| 8 | 10 | | 93.00 | 5.00 | - | 2.00 | - | - |
| | 11 | | 91.25 | 5.75 | - | - | 3.00 | - |
| | 12 | | 95.00 | 5.00 | - | - | - | - |
| 9 | 13 | DT 87-6911 | 91.50 | 6.50 | | | | 2.00 |
| | 14 | | 90.00 | 6.00 | 4.00 | - | - | - |
| | 15 | | 90.00 | 10.00 | - | - | - | - |
| 10 | 16 | | 93.00 | 5.00 | - | 2.00 | - | - |
| | 17 | | 91.25 | 5.75 | - | - | 3.00 | - |
| | 18 | | 95.00 | 5.00 | - | - | - | - |
| 11 | 19 | Bio PSA 7-4301 | 91.50 | 6.50 | | | | 2.00 |
| | 20 | | 90.00 | 6.00 | 4.00 | - | - | - |
| | 21 | | 90.00 | 10.00 | - | - | - | - |
| 12 | 22 | | 93.00 | 5.00 | - | 2.00 | - | - |
| | 23 | | 91.25 | 5.75 | - | - | 3.00 | - |
| | 24 | | 95.00 | 5.00 | - | - | - | - |

The same conditions as in Example 4 were used except for one additional sample which was taken after 1 h.

The permeation results for hydroxyl-modified acrylate adhesives confirm the data obtained in Example 4. The cumulative amount of Tapentadol (API) detected in the permeation cells after 24 h increases steeply after a lag-time of

2 to 3 h. The highest values observed come from DT 87-4287 with a content of API of 10 % (table 9, sample 3). For the samples 1, 2, 4 and 5 containing percutaneous penetration enhancers, only slightly better permeation values in the range of from 300 to 400 μg/cm$^2$ are obtained as compared to sample 6 containing no percutaneous penetration enhancer at a content of API of 5 %. Sample 2 is an exception since its values exceed 500 μg/mL.

The properties of the acrylic-rubber hybrid adhesive DT 87-502A are comparable to those of DT 87-4287. The maximum Tapentadol concentration of 500 to 600 μg/mL after 24 h is observed with sample 9 containing 10 % of API. While the enhancement caused by oleic acid (sample 10) is marginal, dodecanol and isopropyl myristate (IPM) are able to increase the cumulative permeation of API by 10 to 15 % (samples 8 and 11). The fact that sample 7 exhibits an overall skin permeation which is approximately 50 % higher than the one of sample 12 cannot be satisfactorily explained solely by a higher content of API (ca. 30%). If normalized on the concentration of API, Span 60 increases the permeation by more than 20 % (sample 7).

Unlike the samples with an acrylate matrix, the overall depletion of the single samples does not exceed 85% and the mean values vary in the range of 60 to 70 %. As a result, the permeation curves progress more smoothly in the time course since the plateau phase is reached later.

The samples bearing a styrene-butadiene-styrene (SBS) matrix show the least correlation of the skin permeation with the quantity of the API. Differences can mostly be attributed to the type of percutaneous penetration enhancer utilized. This becomes most evident from sample 16 of DT 87-6911 containing 2 % of oleic acid. Due to the protonation of the amine functionality, the solubility in the comparatively unpolar matrix is strongly reduced and thus diffusion is inhibited. A higher content of API is not beneficial in general with respect to the amount of API which permeates through the skin. Although sample 14 contains less API (6.5 %) than sample 15 (10 % API) both display comparable permeation rates after 24 h. It appears that the influence of IPM is crucial for fast permeation through mouse skin whereas a larger API reservoir leads to a constant and in case of sample 15 almost linear release profile. Another advantage of the percutaneous penetration enhancer employed in this experimental design is the reduction of lag time up to 2 h.

The utilization of silicone adhesive Bio PSA 7-4301 appears to be challenging for several reasons. First of all, the precise adjustment of coating thickness is difficult due to the low viscosity of the coating solution. As a consequence, the distribution of the coating thickness is relatively irregular. After one to two days, crystallization of Tapentadol in the laminates is observed. This undesired characteristic usually leads to broad variations in the skin permeation because the interaction of the API with the matrix and percutaneous penetration enhancer is weakened. One exception is sample 22 where only small crystals are found due to partial formation of Tapentadol oleate. Furthermore, the samples of Bio PSA 7-4301 have a strong tack making it difficult to peel off the release liner. The coating compound partially sticks to the backing layer. In summary, silicone adhesives are less suitable for the preparation of Tapentadol samples in the formulations tested.

Example 6: *Analysis of the results*

The experimental design for each adhesive is created with the help of DesignExpert® 7. For evaluation, a simplified quadratic D-optimal mixture design with six runs is chosen. The single components are Tapentadol (A), adhesive (B) and percutaneous penetration enhancer (C) as a general term.

Constraints:

$$5\ \% \leq A \leq 10\ \%$$

$$90\ \% \leq B \leq 95\ \%$$

$$0\ \% \leq C \leq 4\ \%$$

$$A + B + C = 100\ \%$$

Analysis: Polynomial with a linear model

*Patches of DT 87-4287:*

The evaluation of the permeation results shows that percutaneous penetration enhancers in general have little

or no effect on the cumulative amount of API detected in the permeation cells. For optimization, i.e. maximization of the response, an increase of the concentration of the API in the polymer matrix will. positively influence the permeation through mouse skin.

*Patches of DT 87-502A:*

The evaluation of the results for the hybrid adhesive DT 87-502A indicate similar properties as obtained for the acrylate matrix described above. As main criterion the API concentration is identified. Again the influence of the percutaneous penetration enhancers is negligible.

*Patches of DT 87-6911:*

The permeation studies of the SBS patches reveal other characteristics than hybrid or acrylate adhesives with respect to the impact of the percutaneous penetration enhancers. Again, the API content in the dry matrix is the by far most critical factor. Yet the permeation rate may also be facilitated by addition of 2 to 3 % of percutaneous penetration enhancer. A minimum permeation per area of 350 $\mu g/cm^2$ can be obtained with an API content of from 6 to 7 % and an increased percutaneous penetration enhancer content of from 3 to 4 %. In summary, SBS formulations can be considered to be more robust than other classes of adhesives as acceptable permeation values are obtainable over a wider range of ingredient ratio.

*Patches of Bio PSA 7-4301:*

For the silicone formulations, an analysis was not performed due to irregularities of the coating thickness and strong crystallization of the API in the matrix.

## Claims

**1.** A pharmaceutical patch for transdermal administration of the pharmacologically active ingredient Tapentadol in form of its free base, the patch comprising

a) a surface layer,
b) an adhesive layer which comprises a pressure sensitive adhesive and at least a portion of the total amount of the pharmacologically active ingredient that is contained in the pharmaceutical patch, and
c) a removable protective layer,

wherein the adhesive layer is located between the surface layer and the removable protective layer; and
wherein the adhesive layer comprises an acrylate-based pressure sensitive adhesive containing a polymer which is derived from a monomer composition comprising monomer units having at least one hydroxyl functional group.

**2.** The pharmaceutical patch according to claim 1, wherein the concentration of the pharmacologically active ingredient in the adhesive layer is within the range of from 1 to 1,500 $g/m^2$ and/or within the range of from 1 to 20 wt.-%, relative to the total weight of the adhesive layer.

**3.** The pharmaceutical patch according to claim 1 or 2, wherein the adhesive layer comprises a copolymer comprising monomer units originating from monomers A which are selected from $C_{1-18}$-alkyl (meth)acrylates and/or monomers B which are copolymerizable with monomers A.

**4.** The pharmaceutical patch according to claim 3, wherein

(i) monomers A are selected from the group consisting of methyl (meth)acrylate, ethyl (meth)acrylate, propyl (meth)acrylate, butyl (meth)acrylate, pentyl (meth)acrylate, hexyl (meth)acrylate, cyclohexyl (meth)acrylate, octyl (meth)acrylate, isobornyl (meth)acrylate, and mixtures thereof; and/or
(ii) monomers B are selected from the group consisting of 2-hydroxyethyl (meth)-acrylate, glyceryl mono(meth)acrylate, glycidyl (meth)acrylate, acrylamide, N,N-diethyl(meth)acrylamide, 2-ethoxyethyl (meth)acrylate, 2-ethoxyethoxyethyl (meth)acrylate, tetrahydrofuryl (meth)acrylate, vinyl acetate, N-vinyl pyrrolidone and mixtures thereof.

5. The pharmaceutical patch according to any of the preceding claims, wherein the adhesive layer comprises a permeation component which enhances permeation of the pharmacologically active ingredient through human skin.

6. The pharmaceutical patch according to claim 5, wherein the relative weight ratio of the pharmacologically active ingredient to the permeation component is within the range of from 1,000 : 1 to 1 : 1,000.

7. The pharmaceutical patch according to claim 5 or 6, wherein the permeation component comprises an alcohol and/or a fatty acid and/or a fatty acid ester.

8. The pharmaceutical patch according to claim 7, wherein the alcohol is dodecanol, the fatty acid is oleic acid and the fatty acid ester is isopropyl myristate or sorbitan monostearate.

9. The pharmaceutical patch according to any of the preceding claims, which upon application to the human skin provides release of the pharmacologically active ingredient at a rate of at least 1.0 $\mu g \cdot cm^{-2} \cdot h^{-1}$ over a period of at least 6 hours.

**Patentansprüche**

1. Pharmazeutisches Pflaster zur transdermalen Verabreichung des pharmakologischen Wirkstoffs Tapentadol in Form seiner freien Base, wobei das Pflaster Folgendes umfasst:

a) eine Oberflächenschicht,
b) eine Klebeschicht, die einen druckempfindlichen Klebstoff und mindestens einen Teil der Gesamtmenge des pharmakologischen Wirkstoffs, der in dem pharmazeutischen Pflaster enthalten ist, umfasst, und
c) eine ablösbare Schutzschicht,

wobei sich die Klebeschicht zwischen der Oberflächenschicht und der ablösbaren Schutzschicht befindet; und wobei die Klebeschicht einen acrylatbasierten druckempfindlichen Klebstoff enthaltend ein Polymer umfasst, das von einer Monomerzusammensetzung umfassend Monomereinheiten aufweisend mindestens eine funktionelle Hydroxylgruppe abgeleitet ist.

2. Pharmazeutisches Pflaster nach Anspruch 1, wobei die Konzentration des pharmakologischen Wirkstoffs in der Klebeschicht innerhalb des Bereichs von 1 bis 1.500 $g/m^2$ und/oder innerhalb des Bereichs von 1 bis 20 Gew.-%, relativ zum Gesamtgewicht der Klebeschicht, liegt.

3. Pharmazeutisches Pflaster nach Anspruch 1 oder 2, wobei die Klebeschicht ein Copolymer umfassend Monomereinheiten umfasst, die hervorgehen aus Monomeren A, die ausgewählt sind aus $C_{1-18}$-Alkyl(meth)acrylaten, und/oder Monomeren B, die copolymerisierbar mit Monomeren A sind.

4. Pharmazeutisches Pflaster nach Anspruch 3, wobei

(i) Monomere A ausgewählt sind aus der Gruppe bestehend aus Methyl(meth)acrylat, Ethyl(meth)acrylat, Propyl(meth)acrylat, Butyl(meth)acrylat, Pentyl(meth)acrylat, Hexyl(meth)acrylat, Cyclohexyl(meth)acrylat, Octyl(meth)acrylat, Isobornyl(meth)acrylat und Gemischen davon; und/oder
(ii) Monomere B ausgewählt sind aus der Gruppe bestehend aus 2-Hydroxyethyl(meth)acrylat, Glycerylmono(meth)acrylat, Glycidyl(meth)acrylat, Acrylamid, N,N-Diethyl(meth)acrylamid, 2-Ethoxyethyl(meth)acrylat, 2-Ethoxyethoxyethyl(meth)acrylat, Tetrahydrofuryl(meth)acrylat, Vinylacetat, N-Vinylpyrrolidon und Gemischen davon.

5. Pharmazeutisches Pflaster nach einem der vorhergehenden Ansprüche, wobei die Klebeschicht eine Permeationskomponente umfasst, die die Permeation des pharmakologischen Wirkstoffs durch menschliche Haut verbessert.

6. Pharmazeutisches Pflaster nach Anspruch 5, wobei das relative Gewichtsverhältnis des pharmakologischen Wirkstoffs zu der Permeationskomponente innerhalb des Bereichs von 1.000:1 bis 1:1.000 liegt.

7. Pharmazeutisches Pflaster nach Anspruch 5 oder 6, wobei die Permeationskomponente einen Alkohol und/oder eine Fettsäure und/oder einen Fettsäureester umfasst.

8. Pharmazeutisches Pflaster nach Anspruch 7, wobei der Alkohol Dodecanol ist, die Fettsäure Ölsäure ist und der Fettsäureester Isopropylmyristat oder Sorbitanmonostearat ist.

9. Pharmazeutisches Pflaster nach einem der vorhergehenden Ansprüche, der bei Applikation auf der menschlichen Haut für die Freisetzung des pharmakologischen Wirkstoffs mit einer Rate von mindestens 1,0 $\mu g \cdot cm^{-2} \cdot h^{-1}$ über einen Zeitraum von mindestens 6 Stunden sorgt.

**Revendications**

1. Patch pharmaceutique pour l'administration transdermique d'un principe pharmacologiquement actif, le Tapentadol sous la forme de sa base libre, le patch comprenant

    a) une couche superficielle,
    b) une couche adhésive qui comprend un adhésif sensible à la pression et au moins une partie de la quantité totale du principe pharmacologiquement actif qui est contenue dans le patch pharmaceutique, et
    c) une couche protectrice amovible,

    dans lequel la couche adhésive se situe entre la couche superficielle et la couche protectrice amovible ; et dans lequel la couche adhésive comprend un adhésif sensible à la pression à base d'acrylate contenant un polymère qui est dérivé d'une composition de monomères comprenant des motifs monomères ayant au moins un groupe fonctionnel hydroxyle.

2. Patch pharmaceutique selon la revendication 1, dans lequel la concentration du principe pharmacologiquement actif dans la couche adhésive est dans la plage de 1 à 1500 $g/m^2$ et/ou dans la plage de 1 à 20 % en poids, sur la base du poids total de la couche adhésive.

3. Patch pharmaceutique selon la revendication 1 ou 2, dans lequel la couche adhésive comprend un copolymère comprenant des motifs monomères provenant de monomères A qui sont choisis parmi les (méth)acrylates d'alkyle $C_{1-18}$ et/ou de monomères B qui sont copolymérisables avec les monomères A.

4. Patch pharmaceutique selon la revendication 3, dans lequel

    (i) les monomères A sont choisis dans le groupe constitué par le (méth)acrylate de méthyle, le (méth)acrylate d'éthyle, le (méth)acrylate de propyle, le (méth)acrylate de butyle, le (méth)acrylate de pentyle, le (méth)acrylate d'hexyle, le (méth)acrylate de cyclohexyle, le (méth)-acrylate d'octyle, le (méth)acrylate d'isobornyle, et leurs mélanges ; et/ou
    (ii) les monomères B sont choisis dans le groupe constitué par le (méth)acrylate de 2-hydroxy-éthyle, le mono(méth)acrylate de glycéryle, le (méth)acrylate de glycidyle, l'acrylamide, le N,N-diéthyl(méth)acrylamide, le (méth)acrylate de 2-éthoxyéthyle, le (méth)acrylate de 2-éthoxyéthoxyéthyle, le (méth)acrylate de tétrahydrofuryle, l'acétate de vinyle, la N-vinyl pyrrolidone et leurs mélanges.

5. Patch pharmaceutique selon l'une quelconque des revendications précédentes, dans lequel la couche adhésive comprend un composant de perméation qui améliore la perméation du principe pharmacologiquement actif à travers la peau humaine.

6. Patch pharmaceutique selon la revendication 5, dans lequel le rapport en poids relatif du principe pharmacologiquement actif au composant de perméation est dans la plage de 1000:1 à 1:1000.

7. Patch pharmaceutique selon la revendication 5 ou 6, dans lequel le composant de perméation comprend un alcool et/ou un acide gras et/ou un ester d'acide gras.

8. Patch pharmaceutique selon la revendication 7, dans lequel l'alcool est le dodécanol, l'acide gras est l'acide oléique et l'ester d'acide gras est le myristate d'isopropyle ou le monostéarate de sorbitan.

9. Patch pharmaceutique selon l'une quelconque des revendications précédentes, qui après application à la peau humaine permet la libération du principe pharmacologiquement actif à une vitesse d'au moins 1,0 $\mu g.cm^{-2}.h^{-1}$ sur une période d'au moins 6 heures.

Figure 1

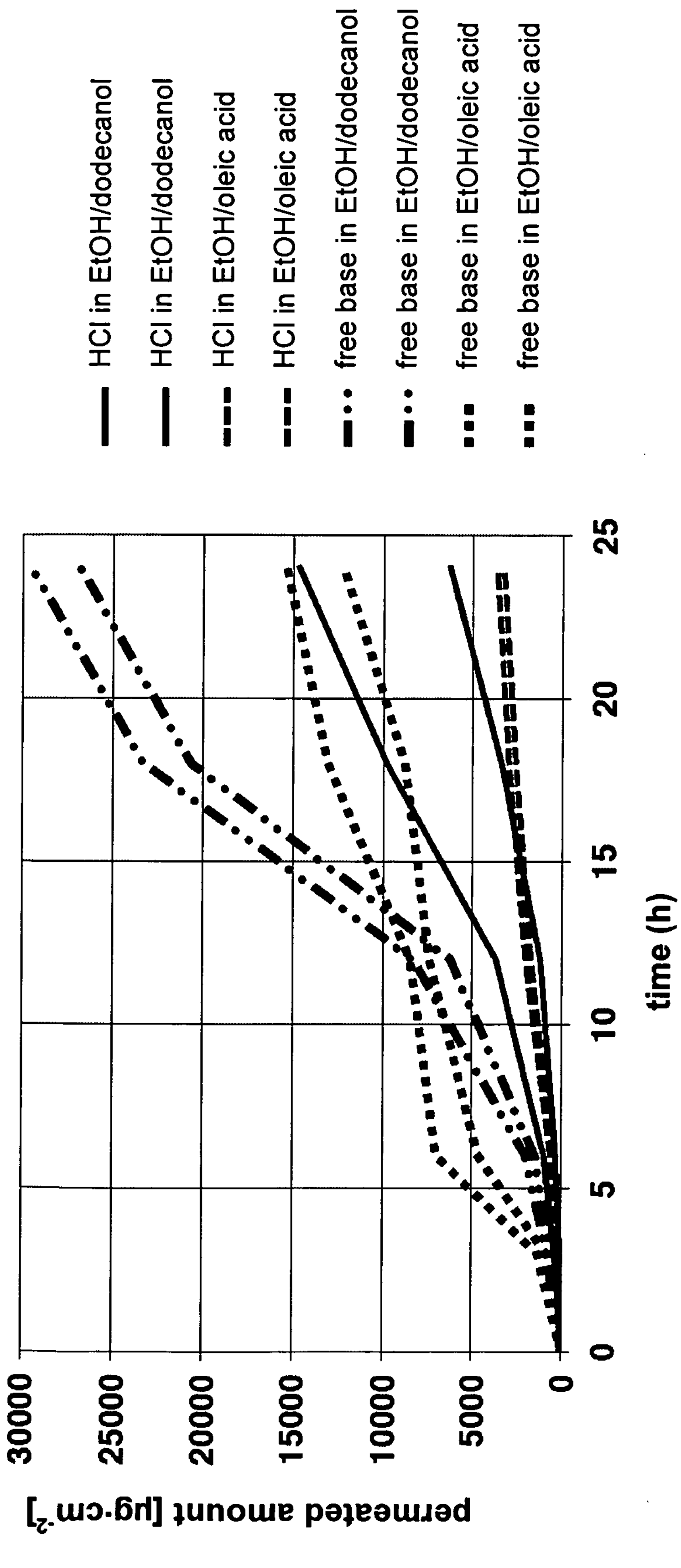
permeated amount [µg·cm-2]
30000
25000
20000
15000
10000
5000
0
0
5
10
15
20
25
time (h)
HCl in EtOH/dodecanol
HCl in EtOH/dodecanol
HCl in EtOH/oleic acid
HCl in EtOH/oleic acid
free base in EtOH/dodecanol
free base in EtOH/dodecanol
free base in EtOH/oleic acid
free base in EtOH/oleic acid

Figure 2

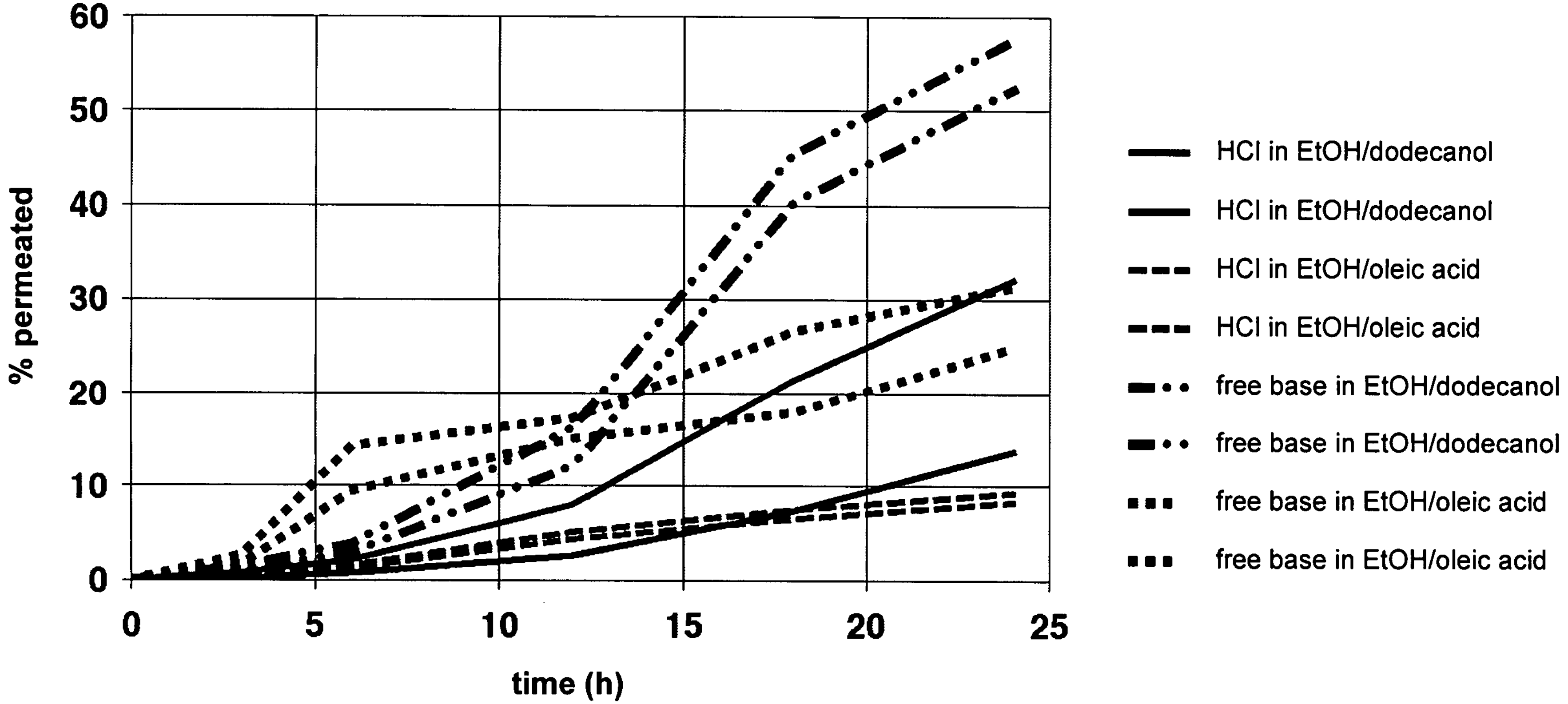
60
50
40
30
20
10
0
% permeated
0
5
10
15
20
25
time (h)
HCl in EtOH/dodecanol
HCl in EtOH/dodecanol
HCl in EtOH/oleic acid
HCl in EtOH/oleic acid
free base in EtOH/dodecanol
free base in EtOH/dodecanol
free base in EtOH/oleic acid
free base in EtOH/oleic acid

Figure 3

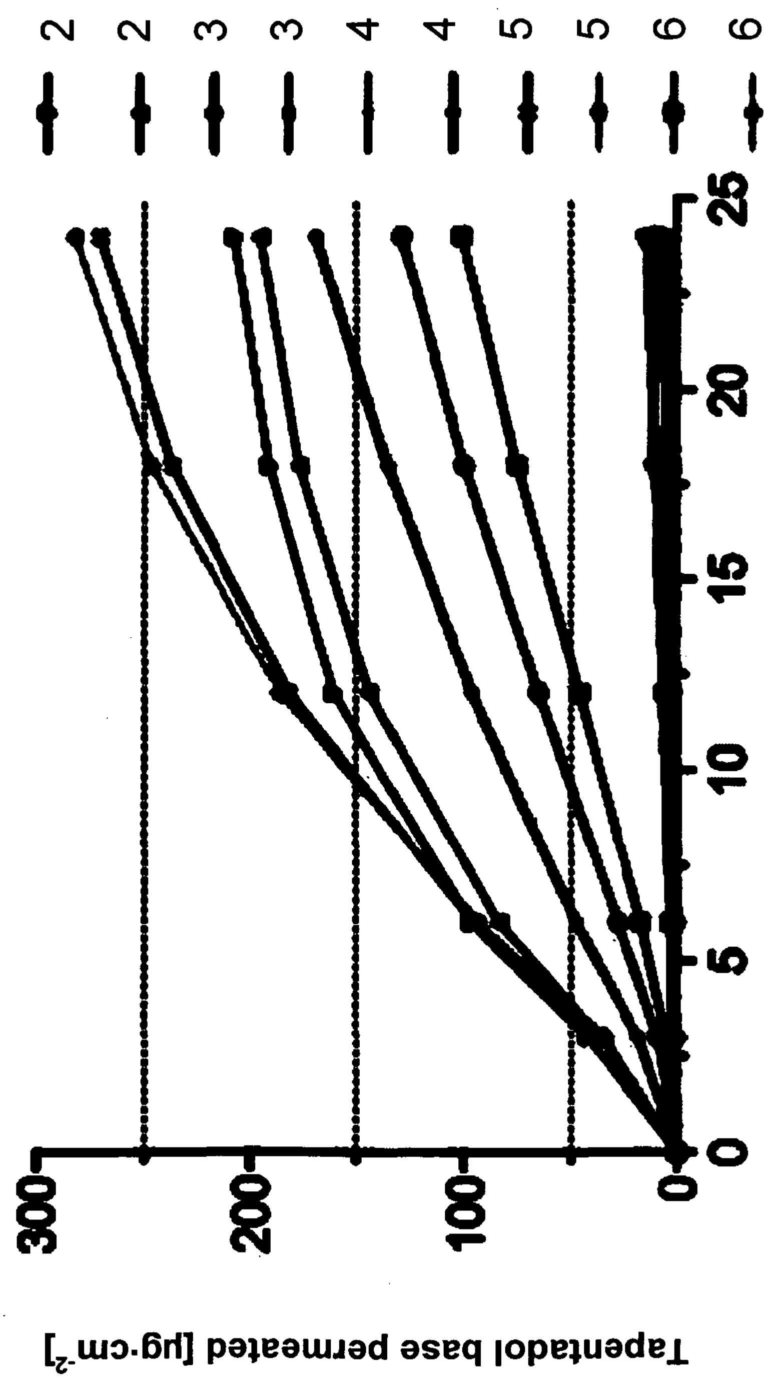
Tapentadol base permeated [µg·cm-2]
time (h)
0
100
200
300
0
5
10
15
20
25
1
1
2
2
3
3
4
4
5
5
6
6

Figure 4

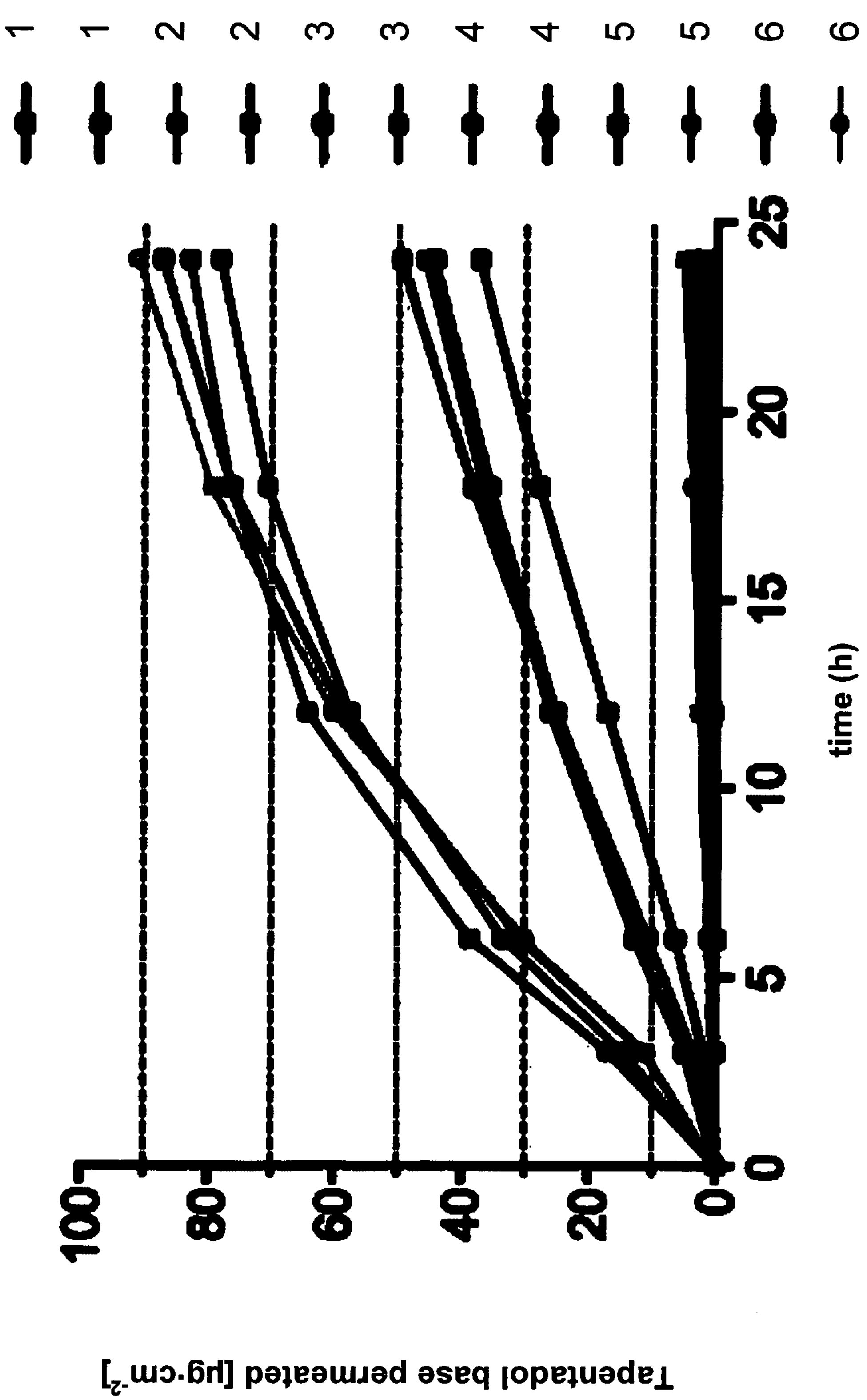
Tapentadol base permeated [µg·cm⁻²]
time (h)
0
20
40
60
80
100
0
5
10
15
20
25
1
1
2
2
3
3
4
4
5
5
6
6

Figure 7

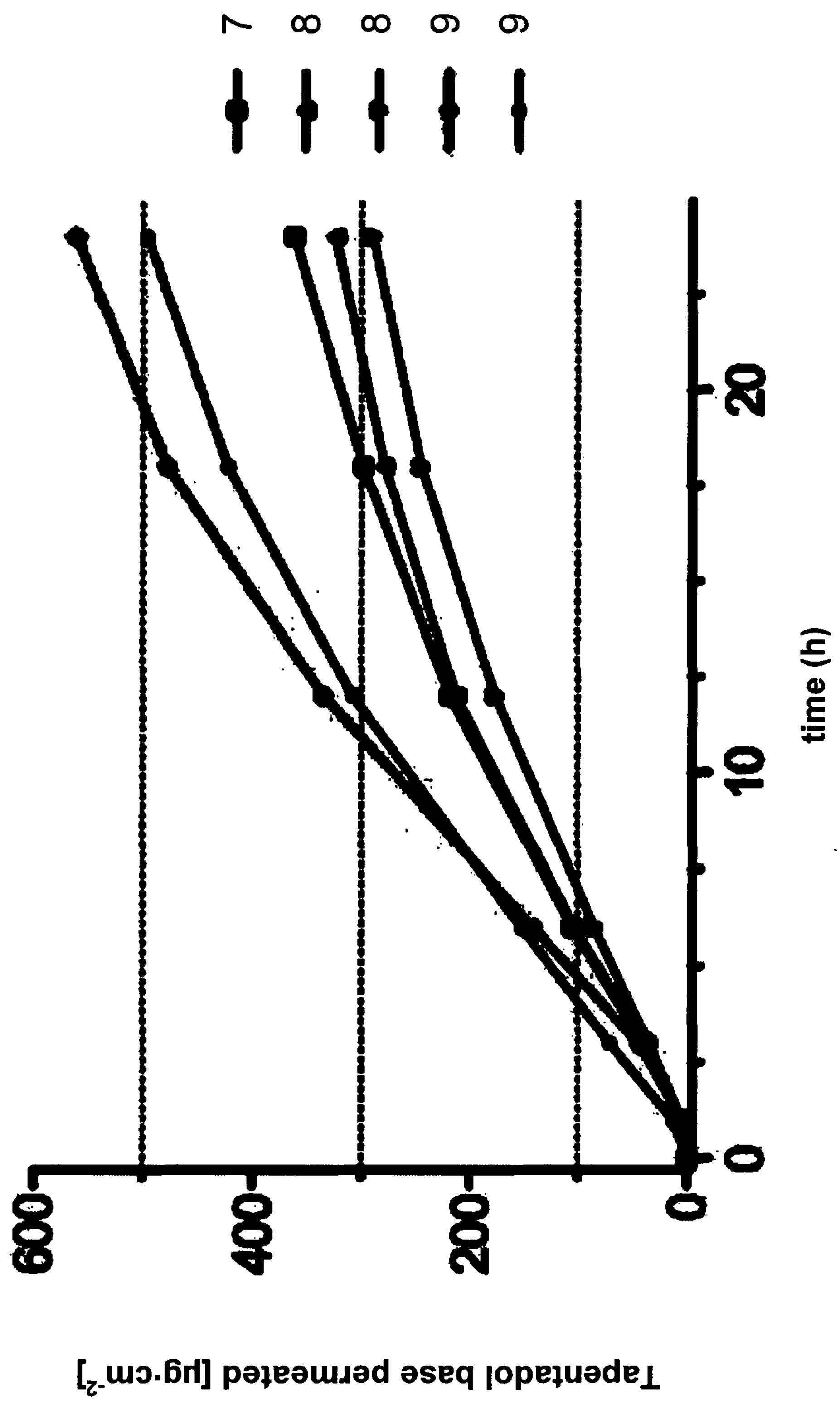
Tapentadol base permeated [µg·cm$^{-2}$]
600
400
200
0
0
10
20
time (h)
7
8
8
9
9

Figure 6

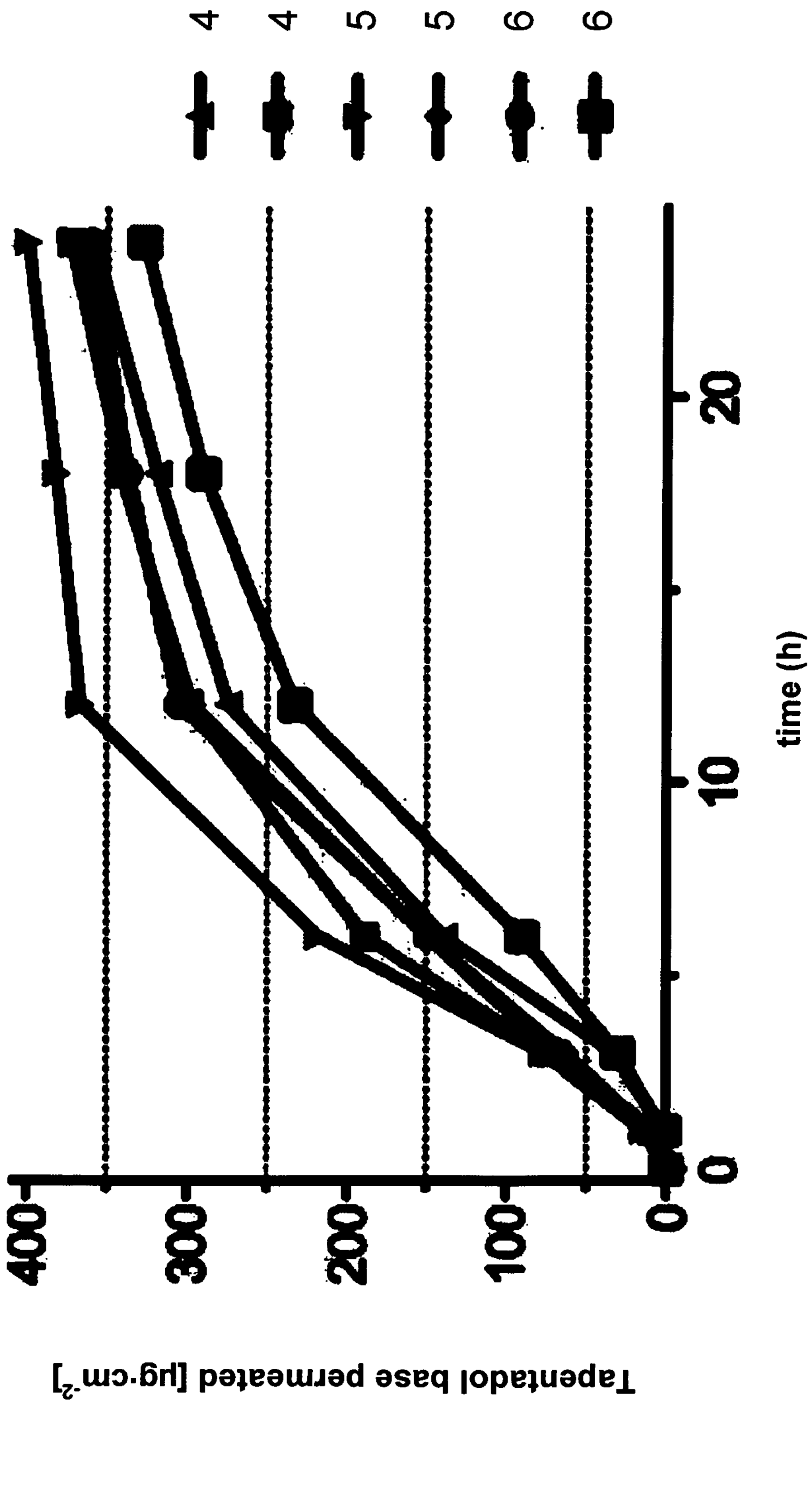
Tapentadol base permeated [µg·cm⁻²]
400
300
200
100
0
0
10
20
time (h)
4
4
5
5
6
6

Figure 5

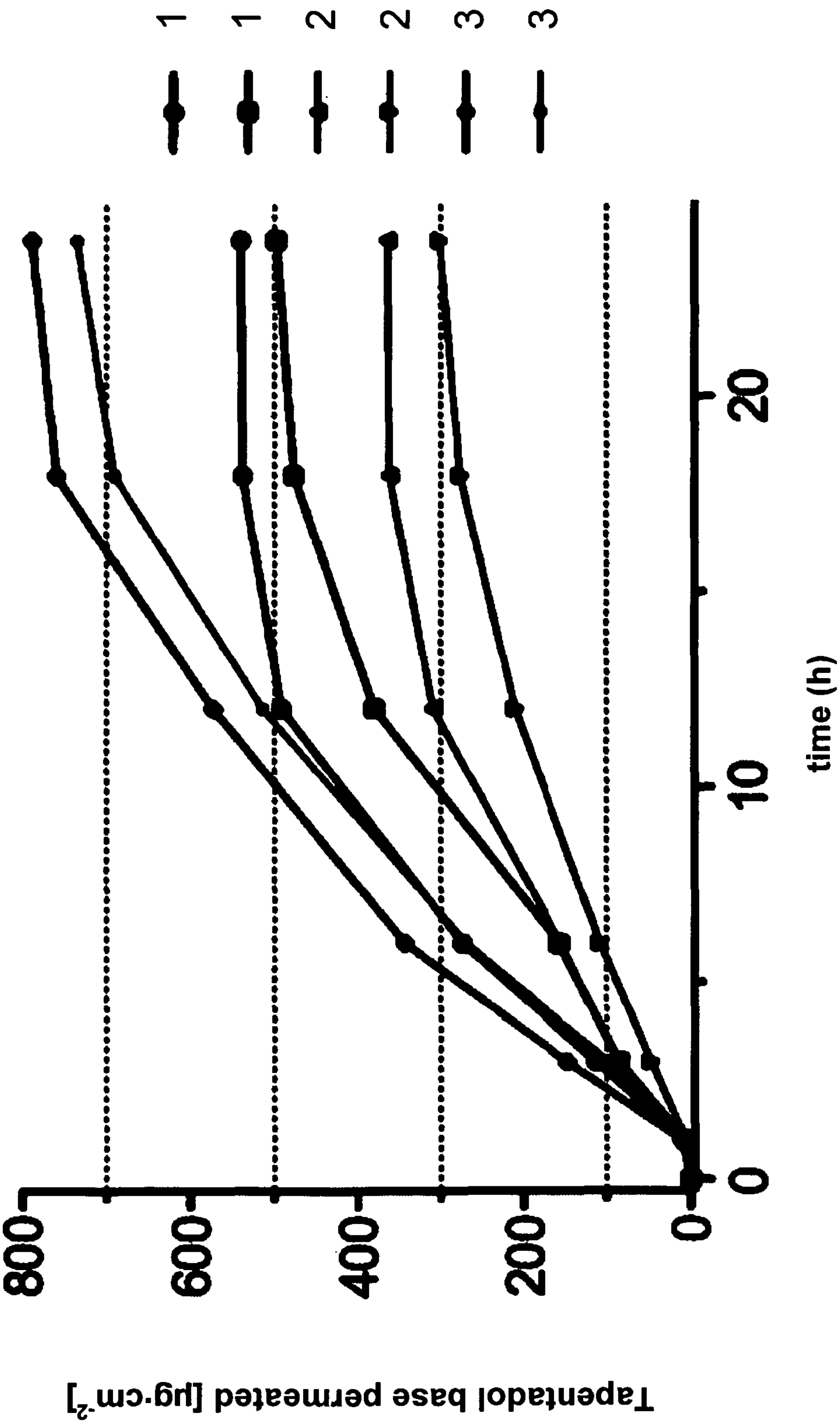
Tapentadol base permeated [µg·cm-2]
800
600
400
200
0
0
10
20
time (h)
1
1
2
2
3
3

Figure 8

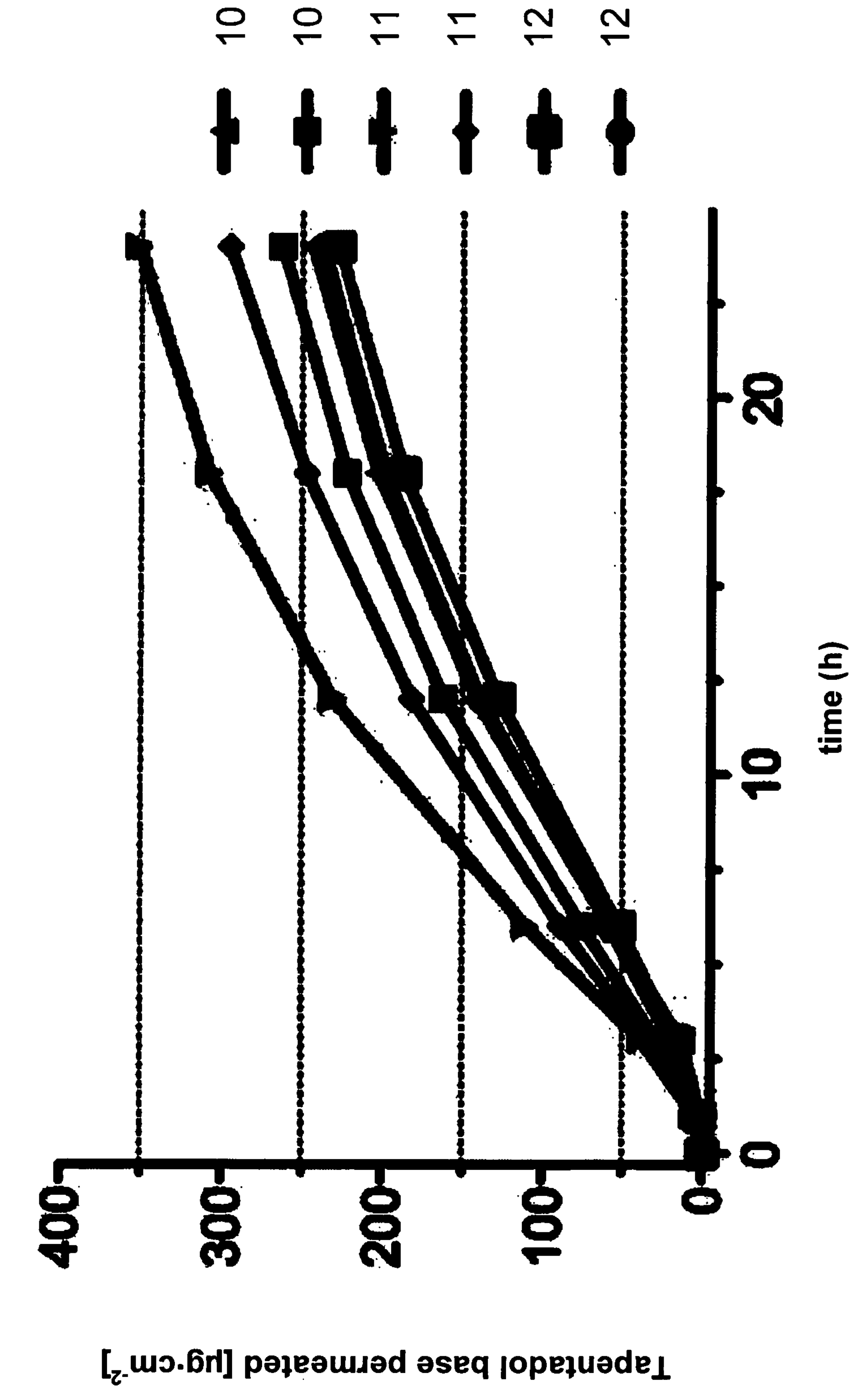
Tapentadol base permeated [µg·cm-2]
400
300
200
100
0
0
10
20
time (h)
10
10
11
11
12
12

Figure 9

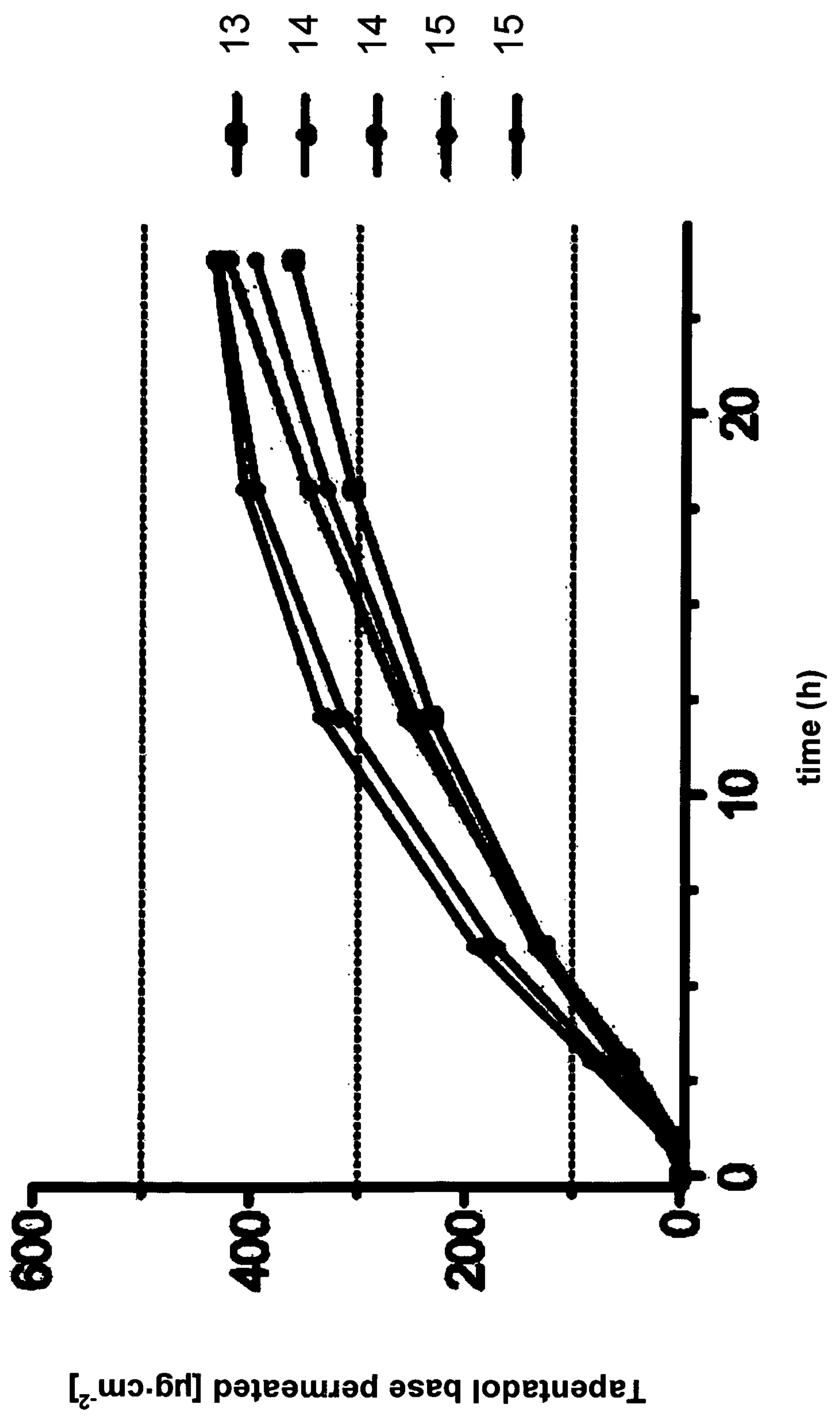

Tapentadol base permeated [µg·cm$^{-2}$]
600
400
200
0
0
10
20
time (h)
13
14
14
15
15

Figure 10

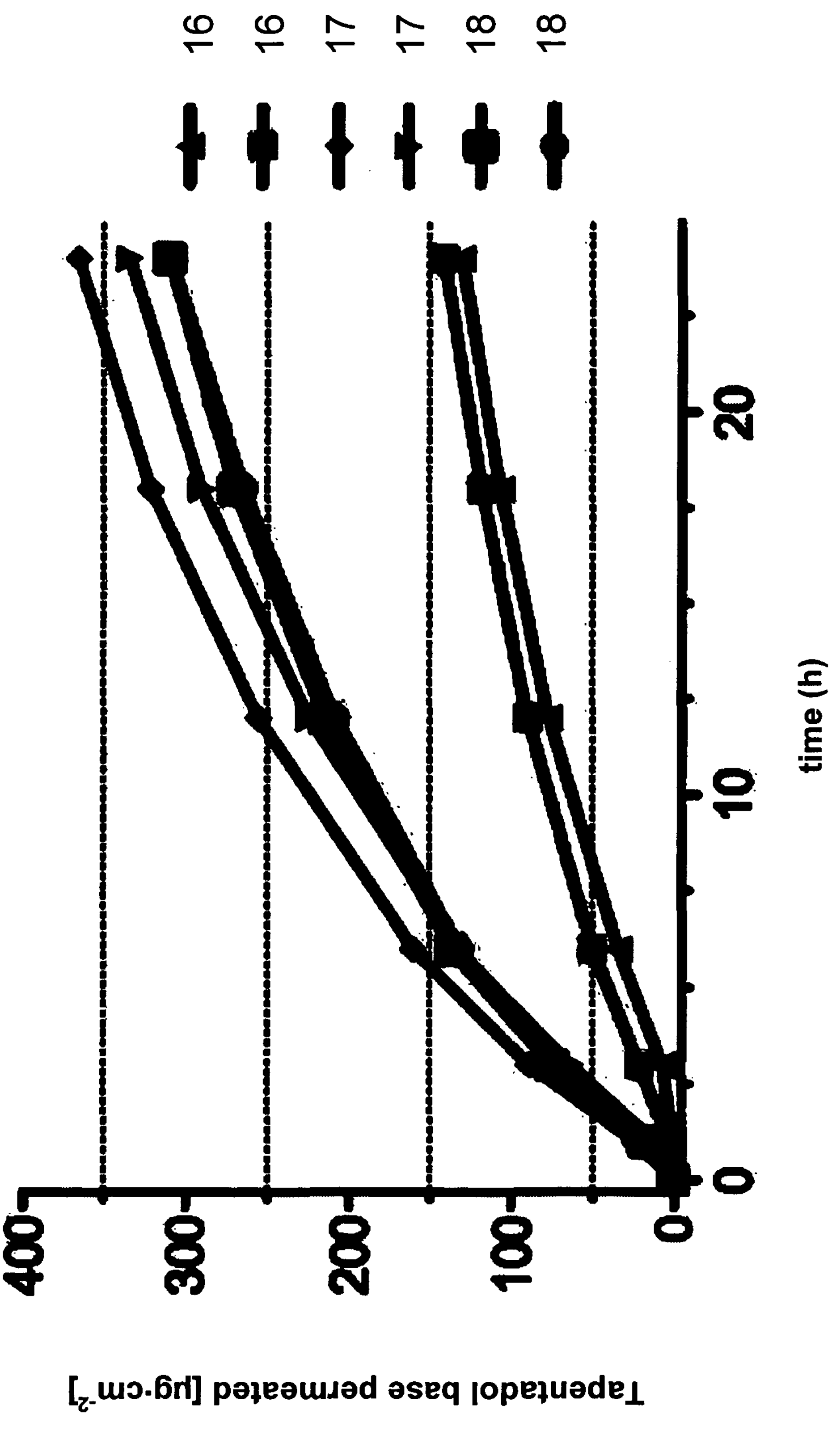
Tapentadol base permeated [$\mu g \cdot cm^{-2}$]
400
300
200
100
0
0
10
20
time (h)
16
16
17
17
18
18

Figure 11

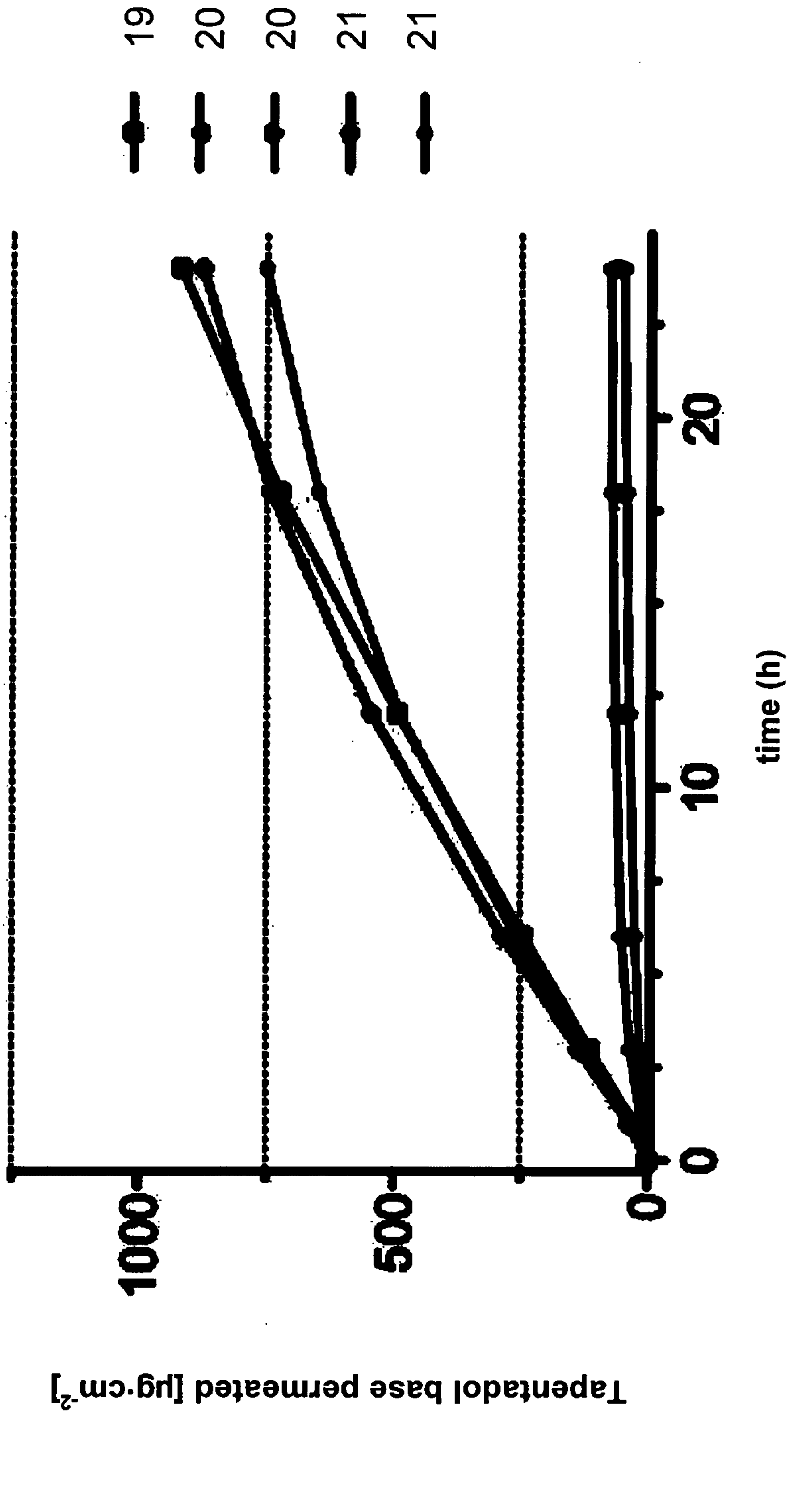
19
20
20
21
21
Tapentadol base permeated [µg·cm-2]
1000
500
0
0
10
20
time (h)

Figure 12

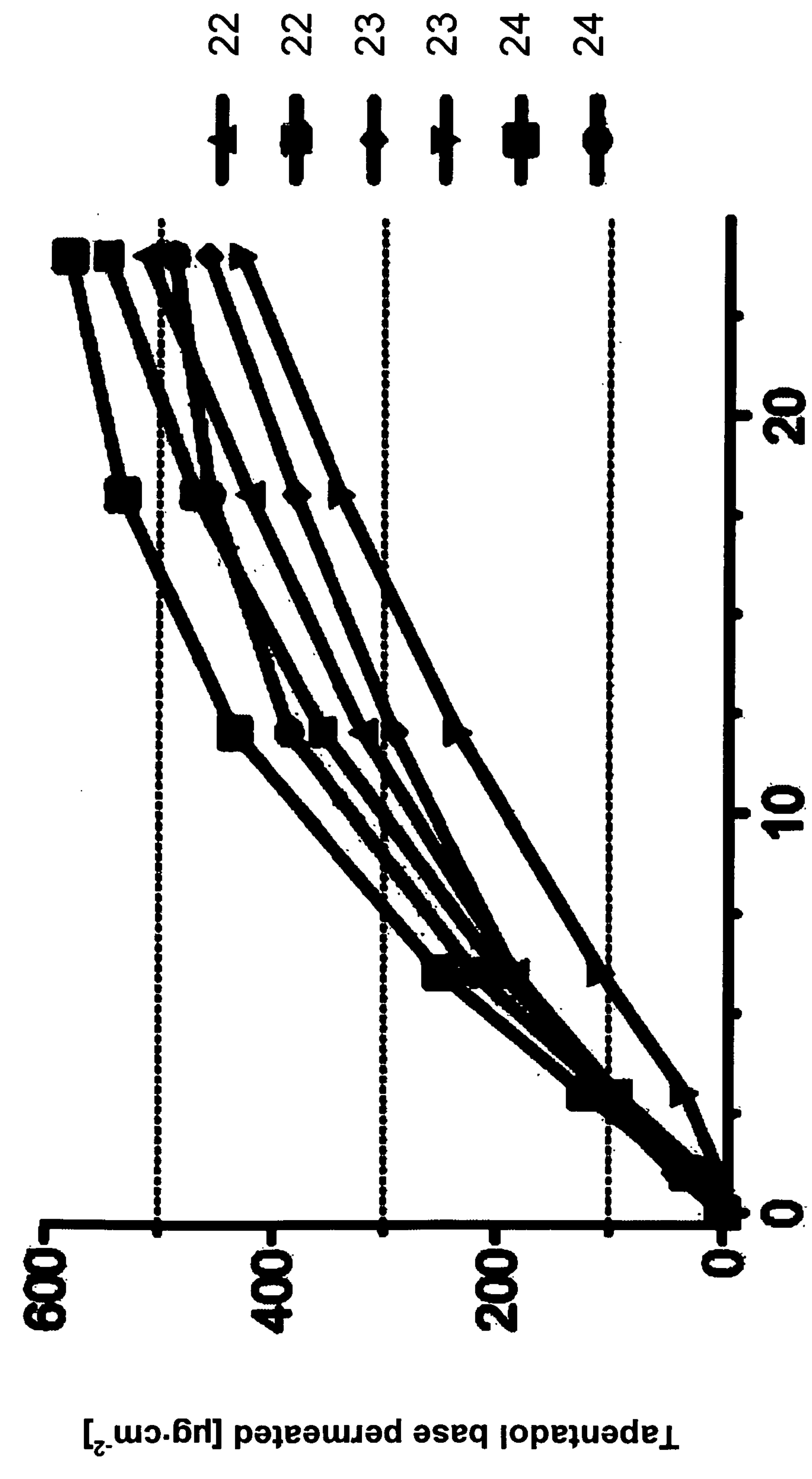
22
22
23
23
24
24
600
400
200
0
0
10
20
time (h)
Tapentadol base permeated [µg·cm$^{-2}$]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 693475 A
- US 20110281855 A
- US 20110244022 A
- US 20072980941 A


### Non-patent literature cited in the description


- **SMITH et al.** Percutaneous Penetration Enhancers. CRC Press, 1995
- **H.A.E. BENSON et al.** Topical and Transdermal Drug Delivery: Principles and Practice. John Wiley & Sons, 2011
- **A.K. BANGA.** Transdermal and Intradermal Delivery of Therapeutic Agents: Application of Physical Technologies. CRC Press Inc, 2011